# EUROPEAN PATENT APPLICATION

(11) **EP 2 390 665 A1**
(43) Date of publication of application: **30.11.2011**
(21) Application number: 10164107.4
(22) Date of filing: 27.05.2010
(51) Int. Cl.: G01N 33/574, C07K 16/40

(54) **Prostate cancer biomarkers**

(71) Applicant: Atlas Antibodies AB, 106 91 Stockholm (SE)
(72) Inventor: Uhlén, Mathias, 182 79 Stocksund (SE); Schwenk, Jochen, 118 53 Stockholm (SE); Nilsson, Peter, 163 52 Spånga (SE)
(74) Representative: Wirén, Anders

(57) **Abstract**

The present disclosure relates to a method of determining whether a mammalian subject belongs to a first or a second group of subjects, wherein the risk of having a prostate cancer, such as an aggressive prostate cancer, is higher in the first group than in the second group. The method comprises the steps of:
a) evaluating an amount of unglycosylated CNDP1 protein in a sample derived from blood, semen or urine from the subject and determining a sample value corresponding to the evaluated amount;
b) comparing the sample value with a predetermined reference value; and if the sample value is lower than or equal to the reference value,
c1) concluding that the subject belongs to the first group; and if the sample value is higher than the reference value,
c2) concluding that the subject belongs to the second group.

Further, the present disclosure relates to a similar method based on the protein HCRTR1 as well as corresponding uses and means useful when carrying out the methods.

SEQUENCE LISTING

<110>  Atlas Antibodies AB

<120>  PROSTATE CANCER BIOMARKERS

<130>  21046657

<160>  17

<170>  PatentIn version 3.3

<210>  1
<211>  102
<212>  PRT
<213>  Homo sapiens

<400>  1

Pro Ala Leu Leu Glu Lys Val Phe Gln Tyr Ile Asp Leu His Gln Asp
1               5                   10                  15

Glu Phe Val Gln Thr Leu Lys Glu Trp Val Ala Ile Glu Ser Asp Ser
            20                  25                  30

Val Gln Pro Val Pro Arg Phe Arg Gln Glu Leu Phe Arg Met Met Ala
        35                  40                  45

Val Ala Ala Asp Thr Leu Gln Arg Leu Gly Ala Arg Val Ala Ser Val
    50                  55                  60

Asp Met Gly Pro Gln Gln Leu Pro Asp Gly Gln Ser Leu Pro Ile Pro
65                  70                  75                  80

Pro Val Ile Leu Ala Glu Leu Gly Ser Asp Pro Thr Lys Gly Thr Val
                85                  90                  95

Cys Phe Tyr Gly His Leu
            100

<210>  2
<211>  131
<212>  PRT
<213>  Homo sapiens

<400>  2

Asp Glu Pro Gly Thr Lys Thr Val Ile Pro Gly Arg Val Ile Gly Lys
1               5                   10                  15

Phe Ser Ile Arg Leu Val Pro His Met Asn Val Ser Ala Val Glu Lys
            20                  25                  30

Gln Val Thr Arg His Leu Glu Asp Val Phe Ser Lys Arg Asn Ser Ser
        35                  40                  45

Asn Lys Met Val Val Ser Met Thr Leu Gly Leu His Pro Trp Ile Ala
    50                  55                  60

Asn Ile Asp Asp Thr Gln Tyr Leu Ala Ala Lys Arg Ala Ile Arg Thr
65                  70                  75                  80

Val Phe Gly Thr Glu Pro Asp Met Ile Arg Asp Gly Ser Thr Ile Pro
                85                  90                  95

Ile Ala Lys Met Phe Gln Glu Ile Val His Lys Ser Val Val Leu Ile
            100                 105                 110

Pro Leu Gly Ala Val Asp Asp Gly Glu His Ser Gln Asn Glu Lys Ile
        115                 120                 125

Asn Arg Trp
    130

<210>  3
<211>  507
<212>  PRT
<213>  Homo sapiens

<400>  3

Met Asp Pro Lys Leu Gly Arg Met Ala Ala Ser Leu Leu Ala Val Leu
1               5                   10                  15

Leu Leu Leu Leu Glu Arg Gly Met Phe Ser Ser Pro Ser Pro Pro Pro
            20                  25                  30

Ala Leu Leu Glu Lys Val Phe Gln Tyr Ile Asp Leu His Gln Asp Glu
        35                  40                  45

Phe Val Gln Thr Leu Lys Glu Trp Val Ala Ile Glu Ser Asp Ser Val
    50                  55                  60

Gln Pro Val Pro Arg Phe Arg Gln Glu Leu Phe Arg Met Met Ala Val
65                  70                  75                  80

Ala Ala Asp Thr Leu Gln Arg Leu Gly Ala Arg Val Ala Ser Val Asp
                85                  90                  95

Met Gly Pro Gln Gln Leu Pro Asp Gly Gln Ser Leu Pro Ile Pro Pro
            100                 105                 110

Ile Ile Leu Ala Glu Leu Gly Ser Asp Pro Thr Lys Gly Thr Val Cys
        115                 120                 125

Phe Tyr Gly His Leu Asp Val Gln Pro Ala Asp Arg Gly Asp Gly Trp
    130                 135                 140

Leu Thr Asp Pro Tyr Val Leu Thr Glu Val Asp Gly Lys Leu Tyr Gly
145                 150                 155                 160

Arg Gly Ala Thr Asp Asn Lys Gly Pro Val Leu Ala Trp Ile Asn Ala

                165                 170                 175

Val Ser Ala Phe Arg Ala Leu Glu Gln Asp Leu Pro Val Asn Ile Lys
            180                 185                 190

Phe Ile Ile Glu Gly Met Glu Glu Ala Gly Ser Val Ala Leu Glu Glu
        195                 200                 205

Leu Val Glu Lys Glu Lys Asp Arg Phe Phe Ser Gly Val Asp Tyr Ile
    210                 215                 220

Val Ile Ser Asp Asn Leu Trp Ile Ser Gln Arg Lys Pro Ala Ile Thr
225                 230                 235                 240

Tyr Gly Thr Arg Gly Asn Ser Tyr Phe Met Val Glu Val Lys Cys Arg
                245                 250                 255

Asp Gln Asp Phe His Ser Gly Thr Phe Gly Gly Ile Leu His Glu Pro
            260                 265                 270

Met Ala Asp Leu Val Ala Leu Leu Gly Ser Leu Val Asp Ser Ser Gly
        275                 280                 285

His Ile Leu Val Pro Gly Ile Tyr Asp Glu Val Val Pro Leu Thr Glu
    290                 295                 300

Glu Glu Ile Asn Thr Tyr Lys Ala Ile His Leu Asp Leu Glu Glu Tyr
305                 310                 315                 320

Arg Asn Ser Ser Arg Val Glu Lys Phe Leu Phe Asp Thr Lys Glu Glu
                325                 330                 335

Ile Leu Met His Leu Trp Arg Tyr Pro Ser Leu Ser Ile His Gly Ile
            340                 345                 350

Glu Gly Ala Phe Asp Glu Pro Gly Thr Lys Thr Val Ile Pro Gly Arg
        355                 360                 365

Val Ile Gly Lys Phe Ser Ile Arg Leu Val Pro His Met Asn Val Ser
    370                 375                 380

Ala Val Glu Lys Gln Val Thr Arg His Leu Glu Asp Val Phe Ser Lys
385                 390                 395                 400

Arg Asn Ser Ser Asn Lys Met Val Val Ser Met Thr Leu Gly Leu His
                405                 410                 415

Pro Trp Ile Ala Asn Ile Asp Asp Thr Gln Tyr Leu Ala Ala Lys Arg
            420                 425                 430

Ala Ile Arg Thr Val Phe Gly Thr Glu Pro Asp Met Ile Arg Asp Gly
        435                 440                 445

Ser Thr Ile Pro Ile Ala Lys Met Phe Gln Glu Ile Val His Lys Ser
    450                 455                 460

Val Val Leu Ile Pro Leu Gly Ala Val Asp Asp Gly Glu His Ser Gln
465                 470                 475                 480

Asn Glu Lys Ile Asn Arg Trp Asn Tyr Ile Glu Gly Thr Lys Leu Phe
                485                 490                 495

Ala Ala Phe Phe Leu Glu Met Ala Gln Leu His
            500                 505

<210>  4
<211>  486
<212>  PRT
<213>  Homo sapiens

<400>  4

Met Ala Ala Ser Leu Leu Ala Val Leu Leu Leu Leu Leu Glu Arg Gly
1               5                   10                  15

Met Phe Ser Ser Pro Ser Pro Pro Pro Ala Leu Leu Glu Lys Val Phe
            20                  25                  30

Gln Tyr Ile Asp Leu His Gln Asp Glu Phe Val Gln Thr Leu Lys Glu
        35                  40                  45

Trp Val Ala Ile Glu Ser Asp Ser Val Gln Pro Val Pro Arg Phe Arg
    50                  55                  60

Gln Glu Leu Phe Arg Met Met Ala Val Ala Ala Asp Thr Leu Gln Arg
65                  70                  75                  80

Leu Pro Asp Gly Gln Ser Leu Pro Ile Pro Pro Ile Ile Leu Ala Glu
                85                  90                  95

Leu Gly Ser Asp Pro Thr Lys Gly Thr Val Cys Phe Tyr Gly His Leu
            100                 105                 110

Asp Val Gln Pro Ala Asp Arg Gly Asp Gly Trp Leu Thr Asp Pro Tyr
        115                 120                 125

Val Leu Thr Glu Val Asp Gly Lys Leu Tyr Gly Arg Gly Ala Thr Asp
    130                 135                 140

Asn Lys Gly Pro Val Leu Ala Trp Ile Asn Ala Val Ser Ala Phe Arg
145                 150                 155                 160

Ala Leu Glu Gln Asp Leu Pro Val Asn Ile Lys Phe Ile Ile Glu Gly
                165                 170                 175

Met Glu Glu Ala Gly Ser Val Ala Leu Glu Glu Leu Val Glu Lys Glu
            180                 185                 190

Lys Asp Arg Phe Phe Ser Gly Val Asp Tyr Ile Val Ile Ser Asp Asn
        195                 200                 205

Leu Trp Ile Ser Gln Arg Lys Pro Ala Ile Thr Tyr Gly Thr Arg Gly
    210                 215                 220

Asn Ser Tyr Phe Met Val Glu Val Lys Cys Arg Asp Gln Asp Phe His
225                 230                 235                 240

Ser Gly Thr Phe Gly Gly Ile Leu His Glu Pro Met Ala Asp Leu Val
                245                 250                 255

Ala Leu Leu Gly Ser Leu Val Asp Ser Ser Gly His Ile Leu Val Pro
            260                 265                 270

Gly Ile Tyr Asp Glu Val Val Pro Leu Thr Glu Glu Glu Ile Asn Thr
        275                 280                 285

Tyr Lys Ala Ile His Leu Asp Leu Glu Glu Tyr Arg Asn Ser Ser Arg
    290                 295                 300

Val Glu Lys Phe Leu Phe Asp Thr Lys Glu Glu Ile Leu Met His Leu
305                 310                 315                 320

Trp Arg Tyr Pro Ser Leu Ser Ile His Gly Ile Glu Gly Ala Phe Asp
                325                 330                 335

Glu Pro Gly Thr Lys Thr Val Ile Pro Gly Arg Val Ile Gly Lys Phe
            340                 345                 350

Ser Ile Arg Leu Val Pro His Met Asn Val Ser Ala Val Glu Lys Gln
        355                 360                 365

Val Thr Arg His Leu Glu Asp Val Phe Ser Lys Arg Asn Ser Ser Asn
    370                 375                 380

Lys Met Val Val Ser Met Thr Leu Gly Leu His Pro Trp Ile Ala Asn
385                 390                 395                 400

Ile Asp Asp Thr Gln Tyr Leu Ala Ala Lys Arg Ala Ile Arg Thr Val
                405                 410                 415

Phe Gly Thr Glu Pro Asp Met Ile Arg Asp Gly Ser Thr Ile Pro Ile
            420                 425                 430

Ala Lys Met Phe Gln Glu Ile Val His Lys Ser Val Val Leu Ile Pro
        435                 440                 445

Leu Gly Ala Val Asp Asp Gly Glu His Ser Gln Asn Glu Lys Ile Asn

450                 455                 460

Arg Trp Asn Tyr Ile Glu Gly Thr Lys Leu Phe Ala Ala Phe Phe Leu
465                 470                 475                 480

Glu Met Ala Gln Leu His
                485

<210>  5
<211>  2215
<212>  DNA
<213>  Homo sapiens

<400>  5
gcattgtctc ttcctctgca gttgagctga atgaatacct ccgaagccgc tttgttctcc      60

agatgtgaat agctccacta taccagcctc gtcttccttc cgggggacaa cgtgggtcag     120

ggcacagaga gatatttaat gtcaccctct tggggctttc atgggactcc ctctgccaca     180

ttttttggag gttgggaaag ttgctagagg cttcagaact ccagcctaat ggatcccaaa     240

ctcgggagaa tggctgcgtc cctgctggct gtgctgctgc tgctgctgga gcgcggcatg     300

ttctcctcac cctccccgcc cccggcgctg ttagagaaag tcttccagta cattgacctc     360

catcaggatg aatttgtgca gacgctgaag gagtgggtgg ccatcgagag cgactctgtc     420

cagcctgtgc ctcgcttcag acaagagctc ttcagaatga tggccgtggc tgcggacacg     480

ctgcagcgcc tgggggcccg tgtggcctcg gtggacatgg gtcctcagca gctgcccgat     540

ggtcagagtc ttccaatacc tcccatcatc ctggccgaac tggggagcga tcccacgaaa     600

ggcaccgtgt gcttctacgg ccacttggac gtgcagcctg ctgaccgggg cgatgggtgg     660

ctcacggacc cctatgtgct gacggaggta gacgggaaac tttatggacg aggagcgacc     720

gacaacaaag gccctgtctt ggcttggatc aatgctgtga gcgccttcag agccctggag     780

caagatcttc ctgtgaatat caaattcatc attgagggga tggaagaggc tggctctgtt     840

gccctggagg aacttgtgga aaaagaaaag gaccgattct tctctggtgt ggactacatt     900

gtaatttcag ataacctgtg gatcagccaa aggaagccag caatcactta cggaacccgg     960

gggaacagct acttcatggt ggaggtgaaa tgcagagacc aggattttca ctcaggaacc    1020

tttggtggca tccttcatga accaatggct gatctggttg ctcttctcgg tagcctggta    1080

gactcgtctg gtcatatcct ggtccctgga atctatgatg aagtggttcc tcttacagaa    1140

gaggaaataa atacatacaa agccatccat ctagacctag aagaataccg gaatagcagc    1200

cgggttgaga aatttctgtt cgatactaag gaggagattc taatgcacct ctggaggtac    1260

ccatctcttt ctattcatgg gatcgagggc gcgtttgatg agcctggaac taaaacagtc    1320

atacctggcc gagttatagg aaaattttca atccgtctag tccctcacat gaatgtgtct    1380

gcggtggaaa aacaggtgac acgacatctt gaagatgtgt tctccaaaag aaatagttcc    1440

aacaagatgg ttgtttccat gactctagga ctacacccgt ggattgcaaa tattgatgac    1500

acccagtatc tcgcagcaaa aagagcgatc agaacagtgt ttggaacaga accagatatg    1560

atccgggatg gatccaccat tccaattgcc aaaatgttcc aggagatcgt ccacaagagc 1620

gtggtgctaa ttccgctggg agctgttgat gatggagaac attcgcagaa tgagaaaatc 1680

aacaggtgga actacataga gggaaccaaa ttatttgctg cctttttctt agagatggcc 1740

cagctccatt aatcacaaga accttctagt ctgatctgat ccactgacag attcacctcc 1800

cccacatccc tagacaggga tggaatgtaa atatccagag aatttgggtc tagtatagta 1860

cattttccct tccatttaaa atgtcttggg atatctggat cagtaataaa atatttcaaa 1920

ggcacagatg ttggaaatgg tttaaggtcc cccactgcac accttcctca agtcatagct 1980

gcttgcagca acttgatttc cccaagtcct gtgcaatagc cccaggattg gattccttca 2040

aaccttttag catatctcca accttgcaat ttgattggca taatcactcc agtttgcttt 2100

ctaggtcctc aagtgctcgt gacacataat cattccatcc aatgatcgcc tttgctttac 2160

cactctttcc ttttatctta ttaataaaaa tgttggtctc caccactgac tacaa 2215

<210> 6
<211> 48
<212> PRT
<213> Homo sapiens

<400> 6

Met Glu Pro Ser Ala Thr Pro Gly Ala Gln Met Gly Val Pro Pro Gly
1               5                   10                  15

Ser Arg Glu Pro Ser Pro Val Pro Pro Asp Tyr Glu Asp Glu Phe Leu
            20                  25                  30

Arg Tyr Leu Trp Arg Asp Tyr Leu Tyr Pro Lys Gln Tyr Glu Trp Val
        35                  40                  45

<210> 7
<211> 389
<212> PRT
<213> Homo sapiens

<400> 7

Met Glu Pro Ser Ala Thr Pro Gly Ala Gln Met Gly Val Pro Pro Gly
1               5                   10                  15

Ser Arg Glu Pro Ser Pro Val Pro Pro Asp Tyr Glu Asp Glu Phe Leu
            20                  25                  30

Arg Tyr Leu Trp Arg Asp Tyr Leu Tyr Pro Lys Gln Tyr Glu Trp Val
        35                  40                  45

Leu Ile Ala Ala Tyr Val Ala Val Phe Val Val Ala Leu Val Gly Asn
    50                  55                  60

Thr Leu Val Cys Leu Ala Val Trp Arg Asn His His Met Arg Thr Val
65                  70                  75                  80

Thr Asn Tyr Phe Ile Val Asn Leu Ser Leu Ala Asp Val Leu Val Thr
                85                  90                  95

Ala Ile Cys Leu Pro Ala Ser Leu Leu Val Asp Ile Thr Glu Ser Trp
            100                 105                 110

Leu Phe Gly His Ala Leu Cys Lys Val Ile Pro Tyr Leu Gln Ala Val
        115                 120                 125

Ser Val Ser Val Ala Val Leu Thr Leu Ser Phe Ile Ala Leu Asp Arg
    130                 135                 140

Trp Tyr Ala Ile Cys His Pro Leu Leu Phe Lys Ser Thr Ala Arg Arg
145                 150                 155                 160

Ala Arg Gly Ser Ile Leu Gly Ile Trp Ala Val Ser Leu Ala Ile Met
                165                 170                 175

Val Pro Gln Ala Ala Val Met Glu Cys Ser Ser Val Leu Pro Glu Leu
            180                 185                 190

Ala Asn Arg Thr Arg Leu Phe Ser Val Cys Asp Glu Arg Trp Ala Asp
        195                 200                 205

Asp Leu Tyr Pro Lys Ile Tyr His Ser Cys Phe Phe Ile Val Thr Tyr
    210                 215                 220

Leu Ala Pro Leu Gly Leu Met Ala Met Ala Tyr Phe Gln Ile Phe Arg
225                 230                 235                 240

Lys Leu Trp Gly Arg Gln Ile Pro Gly Thr Thr Ser Ala Leu Val Arg
                245                 250                 255

Asn Trp Lys Arg Pro Ser Asp Gln Leu Gly Asp Leu Glu Gln Gly Leu
            260                 265                 270

Ser Gly Glu Pro Gln Pro Arg Ala Arg Ala Phe Leu Ala Glu Val Lys
        275                 280                 285

Gln Met Arg Ala Arg Arg Lys Thr Ala Lys Met Leu Met Val Val Leu
    290                 295                 300

Leu Val Phe Ala Leu Cys Tyr Leu Pro Ile Ser Val Leu Asn Val Leu
305                 310                 315                 320

Lys Arg Val Phe Gly Met Phe Arg Gln Ala Ser Asp Arg Glu Ala Val
                325                 330                 335

Tyr Ala Cys Phe Thr Phe Ser His Trp Leu Val Tyr Ala Asn Ser Ala
            340                 345                 350

Ala Asn Pro Ile Ile Tyr Asn Phe Leu Ser Gly Cys Lys Glu Lys Ser

355                 360                 365

Leu Ala Leu Ser Cys Pro Ser Cys Pro Gly His Asp Pro His Leu Ala
    370                 375                 380

Ala Ala Leu Cys Ser
385

<210>  8
<211>  425
<212>  PRT
<213>  Homo sapiens

<400>  8

Met Glu Pro Ser Ala Thr Pro Gly Ala Gln Met Gly Val Pro Pro Gly
1               5                   10                  15

Ser Arg Glu Pro Ser Pro Val Pro Pro Asp Tyr Glu Asp Glu Phe Leu
            20                  25                  30

Arg Tyr Leu Trp Arg Asp Tyr Leu Tyr Pro Lys Gln Tyr Glu Trp Val
        35                  40                  45

Leu Ile Ala Ala Tyr Val Ala Val Phe Val Val Ala Leu Val Gly Asn
    50                  55                  60

Thr Leu Val Cys Leu Ala Val Trp Arg Asn His His Met Arg Thr Val
65                  70                  75                  80

Thr Asn Tyr Phe Ile Val Asn Leu Ser Leu Ala Asp Val Leu Val Thr
                85                  90                  95

Ala Ile Cys Leu Pro Ala Ser Leu Leu Val Asp Ile Thr Glu Ser Trp
            100                 105                 110

Leu Phe Gly His Ala Leu Cys Lys Val Ile Pro Tyr Leu Gln Ala Val
        115                 120                 125

Ser Val Ser Val Ala Val Leu Thr Leu Ser Phe Ile Ala Leu Asp Arg
    130                 135                 140

Trp Tyr Ala Ile Cys His Pro Leu Leu Phe Lys Ser Thr Ala Arg Arg
145                 150                 155                 160

Ala Arg Gly Ser Ile Leu Gly Ile Trp Ala Val Ser Leu Ala Ile Met
                165                 170                 175

Val Pro Gln Ala Ala Val Met Glu Cys Ser Ser Val Leu Pro Glu Leu
            180                 185                 190

Ala Asn Arg Thr Arg Leu Phe Ser Val Cys Asp Glu Arg Trp Ala Asp
        195                 200                 205

Asp Leu Tyr Pro Lys Ile Tyr His Ser Cys Phe Phe Ile Val Thr Tyr
    210                 215                 220

Leu Ala Pro Leu Gly Leu Met Ala Met Ala Tyr Phe Gln Ile Phe Arg
225                 230                 235                 240

Lys Leu Trp Gly Arg Gln Ile Pro Gly Thr Thr Ser Ala Leu Val Arg
                245                 250                 255

Asn Trp Lys Arg Pro Ser Asp Gln Leu Gly Asp Leu Glu Gln Gly Leu
            260                 265                 270

Ser Gly Glu Pro Gln Pro Arg Ala Arg Ala Phe Leu Ala Glu Val Lys
        275                 280                 285

Gln Met Arg Ala Arg Arg Lys Thr Ala Lys Met Leu Met Val Val Leu
    290                 295                 300

Leu Val Phe Ala Leu Cys Tyr Leu Pro Ile Ser Val Leu Asn Val Leu
305                 310                 315                 320

Lys Arg Val Phe Gly Met Phe Arg Gln Ala Ser Asp Arg Glu Ala Val
                325                 330                 335

Tyr Ala Cys Phe Thr Phe Ser His Trp Leu Val Tyr Ala Asn Ser Ala
            340                 345                 350

Ala Asn Pro Ile Ile Tyr Asn Phe Leu Ser Gly Lys Phe Arg Glu Gln
        355                 360                 365

Phe Lys Ala Ala Phe Ser Cys Cys Leu Pro Gly Leu Gly Pro Cys Gly
    370                 375                 380

Ser Leu Lys Ala Pro Ser Pro Arg Ser Ser Ala Ser His Lys Ser Leu
385                 390                 395                 400

Ser Leu Gln Ser Arg Cys Ser Ile Ser Lys Ile Ser Glu His Val Val
                405                 410                 415

Leu Thr Ser Val Thr Thr Val Leu Pro
            420                 425

<210>  9
<211>  1170
<212>  DNA
<213>  Homo sapiens

<400>  9
atggagccct cagccacccc aggggcccag atgggggtcc cccctggcag cagagagccg      60

tcccctgtgc ctccagacta tgaagatgag tttctccgct atctgtggcg cgattatctg     120

tacccaaaac agtatgagtg ggtcctcatc gcagcctatg tggctgtgtt cgtcgtggcc     180

ctggtgggca acacgctggt ctgcctggcc gtgtggcgga accaccacat gaggacagtc 240

accaactact tcattgtcaa cctgtccctg gctgacgttc tggtgactgc tatctgcctg 300

ccggccagcc tgctggtgga catcactgag tcctggctgt tcggccatgc cctctgcaag 360

gtcatcccct atctacaggc tgtgtccgtg tcagtggcag tgctaactct cagcttcatc 420

gccctggacc gctggtatgc catctgccac ccactattgt tcaagagcac agcccggcgg 480

gcccgtggct ccatcctggg catctgggct gtgtcgctgg ccatcatggt gccccaggct 540

gcagtcatgg aatgcagcag tgtgctgcct gagctagcca accgcacacg gctcttctca 600

gtctgtgatg aacgctgggc agatgacctc tatcccaaga tctaccacag ttgcttcttt 660

attgtcacct acctggcccc actgggcctc atggccatgg cctatttcca gatattccgc 720

aagctctggg gccgccagat ccccggcacc acctcagcac tggtgcggaa ctggaagcgc 780

ccctcagacc agctggggga cctggagcag ggcctgagtg gagagcccca gccccgggcc 840

cgcgccttcc tggctgaagt gaagcagatg cgtgcacgga ggaagacagc caagatgctg 900

atggtggtgc tgctggtctt cgccctctgc tacctgccca tcagcgtcct caatgtcctt 960

aagagggtgt tcgggatgtt ccgccaagcc agtgaccgcg aagctgtcta cgcctgcttc 1020

accttctccc actggctggt gtacgccaac agcgctgcca accccatcat ctacaacttc 1080

ctcagtgggt gtaaagagaa gagtctagct ctgtcctgcc catcgtgccc cggccatgac 1140

ccgcaccttg ctgcagctct gtgtagctaa 1170

<210> 10
<211> 19
<212> DNA
<213> Homo sapiens

<400> 10
ccggcgctgt tagagaaag 19

<210> 11
<211> 18
<212> DNA
<213> Homo sapiens

<400> 11
caagtggccg tagaagca 18

<210> 12
<211> 19
<212> DNA
<213> Homo sapiens

<400> 12
ccggcgctgt tagagaaag 19

<210> 13
<211> 18
<212> DNA
<213> Homo sapiens

<400> 13
caagtggccg tagaagca 18

<210>  14
<211>  20
<212>  DNA
<213>  Homo sapiens

<400>  14
gatgagcctg gaactaaaac                                                    20


<210>  15
<211>  22
<212>  DNA
<213>  Homo sapiens

<400>  15
ccacctgttg attttctcat tc                                                 22


<210>  16
<211>  2152
<212>  DNA
<213>  Homo sapiens

<400>  16
gcagttgagc tgaatgaata cctccgaagc cgctttgttc tccagatgtg aatagctcca      60

ctataccagc ctcgtcttcc ttccggggga caacgtgggt cagggcacag agagatattt     120

aatgtcaccc tcttggggct ttcatgggac tccctctgcc acattttttg gaggttggga      180

aagttgctag aggcttcaga actccagcct aatggatccc aaactcggga gaatggctgc     240

gtccctgctg gctgtgctgc tgctgctgct ggagcgcggc atgttctcct caccctcccc     300

gcccccggcg ctgttagaga aagtcttcca gtacattgac ctccatcagg atgaatttgt     360

gcagacgctg aaggagtggg tggccatcga gagcgactct gtccagcctg tgcctcgctt     420

cagacaagag ctcttcagaa tgatggccgt ggctgcggac acgctgcagc gcctgcccga     480

tggtcagagt cttccaatac ctcccatcat cctggccgaa ctggggagcg atcccacgaa     540

aggcaccgtg tgcttctacg gccacttgga cgtgcagcct gctgaccggg gcgatgggtg     600

gctcacggac ccctatgtgc tgacggaggt agacgggaaa ctttatggac gaggagcgac     660

cgacaacaaa ggccctgtct tggcttggat caatgctgtg agcgccttca gagccctgga     720

gcaagatctt cctgtgaata tcaaattcat cattgagggg atggaagagg ctggctctgt     780

tgccctggag gaacttgtgg aaaaagaaaa ggaccgattc ttctctggtg tggactacat     840

tgtaatttca gataacctgt ggatcagcca aaggaagcca gcaatcactt acggaacccg     900

ggggaacagc tacttcatgg tggaggtgaa atgcagagac caggattttc actcaggaac      960

ctttggtggc atccttcatg aaccaatggc tgatctggtt gctcttctcg gtagcctggt    1020

agactcgtct ggtcatatcc tggtccctgg aatctatgat gaagtggttc ctcttacaga    1080

agaggaaata aatacataca aagccatcca tctagaccta gaagaatacc ggaatagcag    1140

ccgggttgag aaatttctgt tcgatactaa ggaggagatt ctaatgcacc tctggaggta    1200

cccatctctt tctattcatg ggatcgaggg cgcgtttgat gagcctggaa ctaaaacagt    1260

catacctggc cgagttatag gaaaattttc aatccgtcta gtccctcaca tgaatgtgtc    1320

tgcggtggaa aaacaggtga cacgacatct tgaagatgtg ttctccaaaa gaaatagttc 1380

caacaagatg gttgtttcca tgactctagg actacacccg tggattgcaa atattgatga 1440

cacccagtat ctcgcagcaa aaagagcgat cagaacagtg tttggaacag aaccagatat 1500

gatccgggat ggatccacca ttccaattgc caaaatgttc caggagatcg tccacaagag 1560

cgtggtgcta attccgctgg gagctgttga tgatggagaa cattcgcaga atgagaaaat 1620

caacaggtgg aactacatag agggaaccaa attatttgct gcctttttct tagagatggc 1680

ccagctccat taatcacaag aaccttctag tctgatctga tccactgaca gattcacctc 1740

ccccacatcc ctagacaggg atggaatgta aatatccaga gaatttgggt ctagtatagt 1800

acattttccc ttccatttaa aatgtcttgg gatatctgga tcagtaataa aatatttcaa 1860

aggcacagat gttggaaatg gtttaaggtc ccccactgca caccttcctc aagtcatagc 1920

tgcttgcagc aacttgattt ccccaagtcc tgtgcaatag ccccaggatt ggattccttc 1980

aaacctttta gcatatctcc aaccttgcaa tttgattggc ataatcactc cagtttgctt 2040

tctaggtcct caagtgctcg tgacacataa tcattccatc caatgatcgc ctttgcttta 2100

ccactctttc cttttatctt attaataaaa atgttggtct ccaccactga ct 2152

<210> 17
<211> 1642
<212> DNA
<213> Homo sapiens

<400> 17
cctcccttca ggaagtttga ggctgagacc cgaaaagacc tgggtgcaag cctccaggca 60

ccctgaaggg agtgggctga gggctggccc aagctccctc ctctccctct gtagagccta 120

ggatgcccct ctgctgcagc ggctcctgag ctcatggagc cctcagccac cccaggggcc 180

cagatggggg tcccccctgg cagcagagag ccgtcccctg tgcctccaga ctatgaagat 240

gagtttctcc gctatctgtg gcgcgattat ctgtacccaa aacagtatga gtgggtcctc 300

atcgcagcct atgtggctgt gttcgtcgtg gccctggtgg gcaacacgct ggtctgcctg 360

gccgtgtggc ggaaccacca catgaggaca gtcaccaact acttcattgt caacctgtcc 420

ctggctgacg ttctggtgac tgctatctgc ctgccggcca gcctgctggt ggacatcact 480

gagtcctggc tgttcggcca tgccctctgc aaggtcatcc cctatctaca ggctgtgtcc 540

gtgtcagtgg cagtgctaac tctcagcttc atcgccctgg accgctggta tgccatctgc 600

cacccactat tgttcaagag cacagcccgg cgggcccgtg gctccatcct gggcatctgg 660

gctgtgtcgc tggccatcat ggtgccccag gctgcagtca tggaatgcag cagtgtgctg 720

cctgagctag ccaaccgcac acggctcttc tcagtctgtg atgaacgctg ggcagatgac 780

ctctatccca agatctacca cagttgcttc tttattgtca cctacctggc cccactgggc 840

ctcatggcca tggcctattt ccagatattc cgcaagctct ggggccgcca gatccccggc 900

accacctcag cactggtgcg gaactggaag cgcccctcag accagctggg ggacctggag 960

cagggcctga gtggagagcc ccagccccgg gcccgcgcct tcctggctga agtgaagcag 1020

atgcgtgcac ggaggaagac agccaagatg ctgatggtgg tgctgctggt cttcgccctc 1080
tgctacctgc ccatcagcgt cctcaatgtc cttaagaggg tgttcgggat gttccgccaa 1140
gccagtgacc gcgaagctgt ctacgcctgc ttcaccttct cccactggct ggtgtacgcc 1200
aacagcgctg ccaaccccat catctacaac ttcctcagtg gcaaattccg ggagcagttt 1260
aaggctgcct tctcctgctg cctgcctggc ctgggtccct gcggctctct gaaggcccct 1320
agtccccgct cctctgccag ccacaagtcc ttgtccttgc agagccgatg ctccatctcc 1380
aaaatctctg agcatgtggt gctcaccagc gtcaccacag tgctgccctg agcgagggct 1440
gccctggagg ctccggctcg ggggatctgc ccctacccct catggaaaga cagctggatg 1500
tggtgaaagg ctgtggcttc agtcctgggt ttctgcctgt gtgactctgg ataagtcact 1560
tcctctgtct gagcttgtgt cccctaagca gggttgatgt gaggattaag catgctgaag 1620
caagtggaaa gctccttgta aa 1642

## Description

### Field of the present disclosure

The present disclosure relates to the field of prostate cancer screening, diagnosis, stratification and treatment.

### Background

### Cancer

Cancer is one of the most common diseases, and a major cause of death in the western world. In general, incidence rates increase with age for most forms of cancer. As human populations continue to live longer, due to an increase of the general health status, cancer may affect an increasing number of individuals. The cause of most common cancer types is still largely unknown, although there is an increasing body of knowledge providing a link between environmental factors (dietary, tobacco smoke, UV radiation etc) as well as genetic factors (germ line mutations in "cancer genes" such as p53, APC, BRCA1, XP etc) and the risk for development of cancer.

No definition of cancer is entirely satisfactory from a cell biological point of view, despite the fact that cancer is essentially a cellular disease and defined as a transformed cell population with net cell growth and anti-social behavior. Malignant transformation represents the transition to a malignant phenotype based on irreversible genetic alterations. Although this has not been formally proven, malignant transformation is believed to take place in one cell, from which a subsequently developed tumor originates (the "clonality of cancer" dogma). Carcinogenesis is the process by which cancer is generated and is generally accepted to include multiple events that ultimately lead to growth of a malignant tumor. This multi-step process includes several rate-limiting steps, such as addition of mutations and possibly also epigenetic events, leading to formation of cancer following stages of precancerous proliferation. The stepwise changes involve accumulation of errors (mutations) in vital regulatory pathways that determine cell division, asocial behavior and cell death. Each of these changes may provide a selective Darwinian growth advantage compared to surrounding cells, resulting in a net growth of the tumor cell population. A malignant tumor does not only necessarily consist of the transformed tumor cells themselves but also surrounding normal cells which act as a supportive stroma. This recruited cancer stroma consists of connective tissue, blood vessels and various other normal cells, e.g., inflammatory cells, which act in concert to supply the transformed tumor cells with signals necessary for continued tumor growth.

The most common forms of cancer arise in somatic cells and are predominantly of epithelial origin, e.g., prostate, breast, colon, urothelium and skin, followed by cancers originating from the hematopoetic lineage, e.g., leukemia and lymphoma, neuroectoderm, e.g., malignant gliomas, and soft tissue tumors, e.g., sarcomas.

### Cancer diagnostics and prognostics

Microscopic evaluation of biopsy material from suspected tumors remains the golden standard for cancer diagnostics. To obtain a firm diagnosis, the tumor tissue is fixated in formalin, histo-processed and paraffin embedded. From the resulting paraffin block, tissue sections can be produced and stained using both histochemical, i.e., hematoxylin-eosin staining, and immunohistochemical (IHC) methods. Also, sections from frozen tissue material may be employed. (IHC allows for the detection of protein expression patterns in tissues and cells using specific antibodies.) The surgical specimen is then evaluated with pathology techniques, including gross and microscopic analysis. This analysis often forms the basis for assigning a specific diagnosis, i.e., classifying the tumor type and grading the degree of malignancy, of a tumor.

Malignant tumors can be categorized into several stages according to classification schemes specific for each cancer type. The most common classification system for solid tumors is the tumor-node-metastasis (TNM) staging system. The T stage describes the local extent of the primary tumor, i.e., how far the tumor has invaded and imposed growth into surrounding tissues, whereas the N stage and M stage describe how the tumor has developed metastases, with the N stage describing spread of tumor to lymph nodes and the M stage describing growth of tumor in other distant organs. Early stages include: T0-1, N0, M0, representing localized tumors with negative lymph nodes. More advanced stages include: T2-4, N0, M0, localized tumors with more widespread growth and T1-4, N1-3, M0, tumors that have metastasized to lymph nodes and T1-4, N1-3, M1, tumors with a metastasis detected in a distant organ. Staging of tumors is often based on several forms of examination, including surgical, radiological and histopathological analyses. In addition to staging, for most tumor types there is also a classification system to grade the level of malignancy. The grading systems rely on morphological assessment of a tumor tissue sample and are based on the microscopic features found in a given tumor. These grading systems may be based on the degree of differentiation, proliferation and atypical appearance of the tumor cells. Examples of generally employed grading systems include Gleason grading for prostatic carcinomas and the Nottingham Histological Grade (NHG) grading for breast carcinomas.

Accurate staging and grading is crucial for a correct diagnosis and may provide an instrument to predict a prognosis. The diagnostic and prognostic information for a specific tumor subsequently determines an adequate therapeutic strategy for a given cancer patient.

### Prostate cancer

World wide, prostate cancer is the second most common form of cancer in men, and in some countries, such as Sweden, it is the most common form. Around 0.7 million new cases of prostate cancer are identified globally each year, and this accounts for more than 11 % of all new cancer cases. However, incidence rates vary vastly across the world. One reason for this is that some countries have screening programs, and in these countries, the number of diagnosed cases has increased dramatically. However, PSA-screening, which is the predominant form of prostate cancer screening, has not been shown to have a clear impact on survival rates.

Also, genetics and diet are factors that influence incidence rates; prostate cancer is least common in South and East Asia, more common in Europe, and most common in black men from the US. The prognosis is relatively good, with a 5-year survival of about 90 %, and even higher in e.g. the USA, where prostate cancer screening may lead to an increase in prostate cancer incidence.

Prostate cancer is predominantly a disease of older men, in Sweden over 60% gets the diagnosis after the age of 70. This cancer is most likely slow growing, and the majority will ultimately die of other causes. However, a problem is that there are no markers to distinguish between the slow growing and more aggressive forms. This makes treatment selection difficult, and every year many people have the prostate surgically removed without a proper cause. Two possible complications associated with prostate removal, incontinence and impotence, make prostatectomy a difficult decision for the patient. Watchful waiting is an alternative strategy, but the risk is higher (see below).

### Prostate cancer diagnostics

Prostate specific antigen (PSA) and its isoforms are currently the only markers recommended for testing of prostate cancer. PSA is specifically expressed in prostate and found upregulated during malignant conditions, but it is also expressed in normal prostate tissue and found upregulated during benign conditions such as hyperplasia and prostatitis. In addition, the normal level of PSA may vary between people, so in order to get a conclusive diagnosis, analysis of tissue from biopsy material is needed. Currently, only 25-30% of all PSA-high cases are confirmed as prostate cancer, thus there is a high false positive rate for prostate cancer detection. A way to improve diagnostic accuracy may be to analyze the free-to-total PSA ratio (f/t PSA). The free PSA molecules are small and readily leak into the blood even at minor disturbances, whereas the presence of PSA bound to a larger molecule, such as a carrier protein, might indicate a more severe prostate tissue disorder. Thus, a low f/t PSA value might be an indicator of a higher risk for prostate cancer than a high PSA-level alone.

Even though PSA is an unreliable marker for prostate cancer, screening programs have nevertheless started in some countries, e.g. the USA. However, the question of screening is controversial, and no clear survival benefit of PSA screening can be stated.

Another diagnostic method is the digital rectal exam (DRE), where a physician palpates the prostate with a finger via the rectum. A problem with this diagnostic method is that when the prostate cancer is large enough to be palpable it is often not curable.

In cases of suspected prostate cancer, such as elevated PSA-levels, a biopsy is generally taken from the prostate, e.g. via the rectum. If using a biopsy gun, several samples may be obtained in seconds. A number of 8-12 biopsy samples may for example be taken. If the biopsy samples are negative for tumor cells, but the PSA-level is still elevated, a second round of biopsies are often performed. If the second round biopsies are also negative, patients are usually monitored regularly regarding PSA-levels, and more biopsies may be taken if the PSA-levels increase further.

In order to diagnose prostate cancer by examining a biopsy, it may be useful to look at biological markers in addition to morphology. High molecular weight cytokeratin and p63 protein expression are rare in prostate cancer and the absence of these proteins may thus indicate tumor tissue. The tumor marker AMACR may also be of interest.

### Treatment of prostate cancer

In Sweden, about half of the patients with prostate cancer are treated with curative intent, either with surgery and/or radiation therapy. Approximately 30 % of those treated with curative intent in Sweden get recurrences. Patients with recurrences may be given hormonal treatment. Hormonal treatment may also be given instead of surgery or radiation therapy as first line treatment. This is normally considered to be an option in cases of metastatic disease, slow growing disease, or if the patient has other life-threatening illnesses. Prostate cancer is, at least initially, dependent on testosterone for growth, and the hormonal treatment may be surgical removal of the testicles or chemical prevention of testosterone from influencing the tumor.

Low-risk prostate cancer patients (see below) may be subject to the watchful waiting strategy, where the patient is closely monitored for disease progression, and is only treated when symptoms appear. The treatment is then often hormonal therapy, see above. The watchful waiting strategy might especially be an option if the life expectancy is less than 10 years, the tumor is slow growing, or the patient has other severe illnesses. If life expectancy is more than 10 years, curative surgery might be an option in the form of radical prostatectomy, where the prostate is removed. Neoadjuvant hormonal therapy may be given before the surgical procedure. A common side effect of radical prostatectomy is impotence. Radiation treatment may also be an option, either in the form of external beam radiation therapy, or in the form of brachytherapy. For low-risk patients, the brachytherapy may be performed by implanting small radioactive "seeds" into the prostate, where they will irradiate the prostate from within. These two therapeutic modalities may also be combined, and neoadjuvant or adjuvant hormonal therapy may also be given in relation with radiation therapy. The risk of impotence is generally lower after radiation therapy than after a surgical procedure.

Intermediate- and high risk patients may be treated by surgery or radiation therapy, but with higher doses than the low-risk patients. Radiation therapy may be given as neoadjuvant and/or adjuvant therapy to radical prostatectomy, and hormonal treatment may be given as neoadjuvant and/or adjuvant therapy to radiation therapy. Other therapeutic options include cryotherapy, whereby the prostate is destroyed by freezing, and high-intensity focused ultrasound, whereby either the tumor or the entire prostate is destroyed by heating.

Patients with metastatic disease may be treated with chemotherapy, and palliative radiation therapy may be given to patients with bone metastases.

### Prognostics and treatment predictive factors

Today, the 5-year survival is approximately 87 % in Sweden. Prostate cancer may, in many cases, be a slow growing disease, where many patients are diagnosed late in life and do not die from their disease.

Prognostic information may be obtained by analyzing biopsy material for a Gleason score, and also by measuring the PSA-level in serum.

The Gleason Grading System is used on biopsy material, in the form of tissue slides, for prognostic information. The Gleason scale is a grading from 1 to 5, where 1 is the lowest grade, representing well differentiated cells that closely resembles normal prostate, and grade 5 represents poorly differentiated cells and tissue without recognizable glands. A Gleason score is then obtained by adding the grade for the two most common tumor patterns, and this score, together with the PSA level (see below), is normally what constitutes the prognostic information.

The PSA-level, an aid in diagnosing prostate cancer, may also provide prognostic information. High levels of PSA indicate a more aggressive cancer.

There are four different stages of prostate cancer. In stage I the cancer is confined to the prostate, has low grade (G), and is not visible by imaging (T1 a, N0, M0, G1), in stage II the cancer is more advanced, but is still confined within the prostate (T1 a-c,or T1-2, N0, M0, any G), in stage III disease the tumor has spread outside the prostate to nearby tissue (T3, N0, M0, any G), and in stage IV the tumor has metastasized to lymph nodes or other parts of the body such as bladder, rectum, bone, liver or lungs (any T, any N, or M1, any G).

Prostate cancer patients may be divided into three risk categories: Low, intermediate, and high risk. The low risk group are identified as those with a PSA concentration of < 10 ng/ml and a Gleason score (GS) of ≤ 6. The intermediate risk group is the patients having PSA levels of 10-20 ng/ml or a GS of 7, and the high risk group is the patients having PSA levels of > 20 ng/ml or a GS of 8-10. In the low and intermediate risk groups survival is high even without treatment with a 10-year survival of 80-90 %.

Nomograms can be used to calculate the estimated risk of the individual patient. The predictions are based on the experience of large groups of patients suffering from cancers at various stages. Various disease factors can be included in the nomograms, such as for example stage, PSA-level, biopsy pathology, use of hormone therapy, and radiation dosage.

### Summary of the present disclosure

The inventors have realized that identification of new diagnostic markers or markers that give a more accurate diagnosis are highly desired and also that the possibility to stratify slow growing from more aggressive cancers would reduce the ongoing overtreatment, e.g. prostatectomy.

It is an object of some aspects of the present disclosure to improve the field of prostate cancer diagnosis and stratification.

Further, it is an object of some aspects of the present disclosure to provide tools useful for such diagnosis and stratification.

The following is a non-limiting and itemized listing of embodiments of the present disclosure, presented for the purpose of providing various features and combinations provided by the invention in certain of its aspects.

### ITEMS

1. Method of determining whether a mammalian subject belongs to a first or a second group of subjects, wherein the risk of having a prostate cancer, such as an aggressive prostate cancer, is higher in the first group than in the second group, comprising the steps of:
   a) evaluating an amount of unglycosylated CNDP1 protein in a sample derived from blood, semen or urine from the subject and determining a sample value corresponding to the evaluated amount;
   b) comparing the sample value with a predetermined reference value; and if the sample value is lower than or equal to the reference value,
   c1) concluding that the subject belongs to the first group; and if the sample value is higher than the reference value,
   c2) concluding that the subject belongs to the second group.
2. Method of determining whether a mammalian subject belongs to a first or a second group of subjects, wherein the risk of having a prostate cancer, such as an aggressive prostate cancer, is higher in the first group than in the second group, comprising the steps of:
   a) quantifying the degree of glycosylation of CNDP1 protein in a sample derived from blood, semen or urine from the subject;
   b) comparing the degree of glycosylation of step a) with a predetermined reference degree of glycosylation; and if the degree of glycosylation of step a) is higher than or equal to the reference,
   c1) concluding that the subject belongs to the first group; and if the degree of glycosylation of step a) is lower than the reference,
   c2) concluding that the subject belongs to the second group.
3. Method according to item 1 or 2, wherein an aggressive prostate cancer is defined as a metastasizing prostate cancer, a prostate cancer showing a Gleason sum of ≥ 8 and/or a prostate cancer from a subject having a PSA level of above 50 ng/ml.
4. Method of detecting a prostate cancer in a mammalian subject, comprising the step of:
   a) evaluating an amount of unglycosylated CNDP1 protein in a sample derived from blood, semen or urine from the subject and determining a sample value corresponding to the evaluated amount;
   b) comparing the sample value with a predetermined reference value; and if the sample value is lower than or equal to the reference value,
   c) examining at least one prostate tissue biopsy from the subject for the presence of tumor cells.
5. Method of selecting a mammalian subject for a prostate tissue examination, comprising the steps of:
   a) evaluating an amount of unglycosylated CNDP1 protein in a sample derived from blood, semen or urine from the subject and determining a sample value corresponding to the evaluated amount;
   b) comparing the sample value with a predetermined reference value; and if the sample value is lower than or equal to the reference value,
   c) selecting the subject for the prostate tissue examination.
6. Method according to item 5, further comprising the step:
   d) performing the prostate tissue examination.
7. Method according to item 5 or 6, wherein the prostate tissue examination comprises examination of at least one prostate tissue biopsy from the subject.
8. Method according to any one of items 5-7, wherein the prostate tissue examination comprises performing at least one prostate tissue biopsy, such as a transrectal or transperineal biopsy, on the subject.
9. Method according to any one of items 5-8, wherein the prostate tissue examination comprises morphological and/or immunohistochemical examination.
10. Method according to any one of items 5-9, wherein the prostate tissue examination comprises examination for the presence of tumor cells and/or a Gleason score.
11. Method for determining whether a mammalian subject belongs to a first or a second group, wherein the prognosis of subjects of the first group is worse than the prognosis of subjects of the second group, comprising the steps of:
   a) evaluating an amount of unglycosylated CNDP1 protein in a sample derived from blood, semen or urine from the subject and determining a sample value corresponding to the evaluated amount;
   b) comparing the sample value with a predetermined reference value; and if the sample value is lower than or equal to the reference value,
   c1) concluding that the subject belongs to the first group; and if the sample value is higher than the reference value,
   c2) concluding that the subject belongs to the second group.
12. Method according to claim 11, in which the subject has prostate cancer.
13. Method for determining whether a mammalian subject is not in need of a prostate cancer treatment regimen, comprising the steps of:
   a) evaluating an amount of unglycosylated CNDP1 protein in a sample derived from blood, semen or urine from the subject and determining a sample value corresponding to the evaluated amount;
   b) comparing the sample value with a predetermined reference value; and if the sample value is higher than the reference value,
   c) concluding that the subject is not in need of the prostate cancer treatment regimen.
14. Non-treatment strategy method for a mammalian subject, comprising the steps of:
   a) evaluating an amount of unglycosylated CNDP1 protein in a sample derived from blood, semen or urine from the subject and determining a sample value corresponding to the evaluated amount;
   b) comparing the sample value with a predetermined reference value; and if the sample value is higher than the reference value,
   c) refraining from treating the subject with a prostate cancer treatment regimen.
15. Method of treatment of a mammalian subject having a prostate cancer, comprising the steps of:
   a) evaluating an amount of unglycosylated CNDP1 protein in a sample derived from blood, semen or urine from the subject and determining a sample value corresponding to the evaluated amount;
   b) comparing the sample value with a predetermined reference value; and if the sample value is lower than or equal to the reference value,
   c) treating the subject with a prostate cancer treatment regimen.
16. Method according to any one of items 13-15, wherein the prostate cancer treatment regimen is selected from the group consisting of surgery, radiation therapy, hormone therapy , cryosurgery, chemotherapy, immunotherapy, high-intensity focused ultrasound and combinations thereof.
17. Method according to any one of the preceding items, wherein the sample is an optionally diluted serum or plasma sample.
18. Method according to any one of the preceding items, wherein step a) comprises contacting the sample with an affinity ligand that is, under the conditions of the contact, capable of selective interaction with the unglycosylated CNDP1 protein.
19. Method according to item 18, wherein the affinity ligand is capable of selective interaction with a peptide whose amino acid sequence consists of SEQ ID NO:1 or SEQ ID NO:2.
20. Method according to item 18 or 19, further comprising quantifying the interaction between the affinity ligand and the unglycosylated form of CNDP1 to obtain the evaluated amount.
21. Method according to any one of the preceding items, wherein step a) comprises a sandwich assay.
22. Method according to item 21, wherein the sandwich assay is an ELISA sandwich assay.
23. Method according to any one of the preceding items, wherein the subject is a male human.
24. CNDP1 protein fragment which consists of 150 amino acids or less, such as 135 amino acids or less, and comprises or consists of amino acid sequence SEQ ID NO:1 or 2.
25. Use *ex vivo* of an unglycosylated CNDP1 protein as a diagnostic marker for prostate cancer.
26. Use according to item 25, wherein the prostate cancer is aggressive prostate cancer.
27. Use according to item 25 or 26, wherein the unglycosylated CNDP1 protein is provided in a blood-derived sample.
28. Use *ex vivo* of an unglycosylated CNDP1 protein, or an antigenically active fragment thereof, for the selection or purification of a diagnostic agent for prostate cancer, such as aggressive prostate cancer.
29. Use of an unglycosylated CNDP1 protein, or an antigenically active fragment thereof, for the production of a diagnostic agent for prostate cancer, such as aggressive prostate cancer.
30. Use according to item 28 or 29, wherein the diagnostic agent is an affinity ligand capable of selective interaction with the unglycosylated CNDP1 protein.
31. Use according to any one of items 25-30, wherein the unglycosylated CNDP1 protein comprises or consists of amino acid sequence SEQ ID NO:3 or 4.
32. Use of an antigenically active fragment according to any one of items 28-30, which fragment is a CNDP1 protein fragment according to item 24.
33. Affinity ligand capable of selective interaction with the unglycosylated CNDP1 protein.
34. Affinity ligand according to item 33, wherein the unglycosylated CNDP1 protein comprises or consists of amino acid sequence SEQ ID NO:3 or 4.
35. Affinity ligand according to item 33 capable of selective interaction with a fragment consisting of amino acid sequence SEQ ID NO:1 or 2.
36. Affinity ligand according to any one of items 33-35, which is capable of selective interaction with the unglycosylated form of CNDP1 in optionally diluted serum or plasma.
37. Affinity ligand according to any one of items 33-36, which is immobilized onto a solid support suitable for a sandwich assay or bound to a bead.
38. Use *ex vivo* of an affinity ligand according to any one of items 33-37 as a diagnostic agent for prostate cancer, such as aggressive prostate cancer.
39. Kit comprising:
   a) a first affinity ligand capable of selective interaction with an unglycosylated CNDP1 protein;
   b) a quantifiable second affinity ligand capable of interaction with the unglycosylated CNDP1 protein when bound to the first affinity ligand; and optionally
   c) a solid support on which the first affinity ligand is immobilized or which is prepared for immobilization of the first affinity ligand.
40. Kit according to item 39, wherein the quantifiable second affinity ligand is capable of selective interaction with the unglycosylated CNDP1 protein when bound to the first affinity ligand.
41. Kit according to item 39 or 40, wherein the first affinity ligand interacts with a CNDP1 amino acid sequence which is different from the CNDP1 amino acid sequence which the quantifiable second affinity ligand interacts with.
42. Kit according to any one of items 39-41, wherein the first affinity ligand is capable of selective interaction with a peptide whose amino acid sequence consist of one of SEQ ID NO:1 and SEQ ID NO:2.
43. Kit according to item 42, wherein the quantifiable second affinity ligand is capable of selective interaction with a peptide whose amino acid sequence consist of the other one of SEQ ID NO:1 and SEQ ID NO:2.
44. Kit according to any one of items 39-43, wherein the quantifiable second affinity ligand is labeled with a quantifiable label.
45. Kit according to any one of items 39-44, further comprising an agent capable of binding the quantifiable second affinity ligand, which agent is labeled with a quantifiable label.
46. Kit according to item 45, wherein the quantifiable second affinity ligand is an antibody and the agent is an antibody capable of binding the Fc region of the quantifiable second affinity ligand.
47. Kit according to any one of items 44-46, wherein the quantifiable label is selected from the group consisting of fluorescent dyes and metals, chromophoric dyes, chemiluminescent compounds and bioluminescent proteins, enzymes, radioisotopes, particles and quantum dots.
48. Kit according to item 47, in which the quantifiable label is an enzyme, further comprising a chromogenic ELISA substrate.
49. Kit according to item 47 or 48, wherein the quantifiable label is an enzyme selected from horseradish peroxidase, alkaline phaosphatase, beta-D-galactosidase and beta-lactamase.
50. Kit according to anyone of items 39-49, wherein the first affinity ligand is an antibody.
51. Kit according to anyone of items 39-50, wherein the quantifiable second affinity ligand is an antibody.
52. Kit according to anyone of items 39-51, wherein the solid support comprises wells in which the first affinity ligand is immobilized or which are prepared for immobilization of the first affinity ligand.
53. Method of determining whether a mammalian subject belongs to a first or a second group of subjects, wherein the risk of having a prostate cancer is higher in the first group than in the second group, comprising the steps of:
   a) evaluating an amount of HCRTR1 protein in a sample derived from blood, semen or urine from the subject and determining a sample value corresponding to the evaluated amount;
   b) comparing the sample value with a predetermined reference value; and if the sample value is lower than or equal to the reference value,
   c1) concluding that the subject belongs to the first group; and if the sample value is higher than the reference value,
   c2) concluding that the subject belongs to the second group.
54. Method of detecting a prostate cancer in a mammalian subject, comprising the step of:
   a) evaluating an amount of HCRTR1 protein in a sample derived from blood, semen or urine from the subject and determining a sample value corresponding to the evaluated amount;
   b) comparing the sample value with a predetermined reference value; and if the sample value is lower than or equal to the reference value,
   c) examining at least one prostate tissue biopsy from the subject for the presence of tumor cells.
55. Method of selecting a mammalian subject for a prostate tissue examination, comprising the steps of:
   a) evaluating an amount of HCRTR1 protein in a sample derived from blood, semen or urine from the subject and determining a sample value corresponding to the evaluated amount;
   b) comparing the sample value with a predetermined reference value; and if the sample value is lower than or equal to the reference value,
   c) selecting the subject for the prostate tissue examination.
56. Method according to item 55, further comprising the step:
   d) performing the prostate tissue examination.
57. Method according to item 55 or 56, wherein the prostate tissue examination comprises examination of at least one prostate tissue biopsy from the subject.
58. Method according to any one of items 55-57, wherein the prostate tissue examination comprises performing at least one prostate tissue biopsy, such as a transrectal or transperineal biopsy, on the subject.
59. Method according to any one of items 55-58, wherein the prostate tissue examination comprises morphological and/or immunohistochemical examination.
60. Method according to any one of items 55-59, wherein the prostate tissue examination comprises examination for the presence of tumor cells and/or a Gleason score.
61. Method for determining whether a mammalian subject is not in need of a prostate cancer treatment regimen, comprising the steps of:
   a) evaluating an amount of HCRTR1 protein in a sample derived from blood, semen or urine from the subject and determining a sample value corresponding to the evaluated amount;
   b) comparing the sample value with a predetermined reference value; and if the sample value is higher than the reference value,
   c) concluding that the subject is not in need of the prostate cancer treatment regimen.
62. Non-treatment strategy method for a mammalian subject, comprising the steps of:
   a) evaluating an amount of HCRTR1 protein in a sample derived from blood, semen or urine from the subject and determining a sample value corresponding to the evaluated amount;
   b) comparing the sample value with a predetermined reference value; and if the sample value is higher than the reference value,
   c) refraining from treating the subject with a prostate cancer treatment regimen.
63. Method of treatment of a mammalian subject having a prostate cancer, comprising the steps of:
   a) evaluating an amount of HCRTR1 protein in a sample derived from blood, semen or urine from the subject and determining a sample value corresponding to the evaluated amount;
   b) comparing the sample value with a predetermined reference value; and if the sample value is lower than or equal to the reference value,
   c) treating the subject with a prostate cancer treatment regimen.
64. Method according to any one of items 61-63, wherein the prostate cancer treatment regimen is selected from the group consisting of surgery, radiation therapy, hormone therapy , cryosurgery, chemotherapy, immunotherapy, high-intensity focused ultrasound and combinations thereof.
65. Method according to any one of items 53-64, wherein the sample is an optionally diluted serum or plasma sample.
66. Method according to any one of items 53-65, wherein step a) comprises contacting the sample with an affinity ligand that is, under the conditions of the contact, capable of selective interaction with the HCRTR1 protein.
67. Method according to item 66, wherein the affinity ligand is capable of selective interaction with a peptide whose amino acid sequence consists of SEQ ID NO:6.
68. Method according to item 66 or 67, further comprising quantifying the interaction between the affinity ligand and the HCRTR1 protein to obtain the evaluated amount.
69. Method according to any one of items 53-68, wherein step a) comprises a sandwich assay.
70. Method according to any one of item 69, wherein the sandwich assay is an ELISA sandwich assay.
71. Method according to any one of the preceding items, wherein the subject is a male human.
72. HCRTR1 protein fragment which consists of 80 amino acids or less, such as 60 amino acids or less, and comprises or consists of amino acid sequence SEQ ID NO:6.
73. Use *ex vivo* of an HCRTR1 protein as a diagnostic marker for prostate cancer.
74. Use according to item 73, wherein the HCRTR1 protein is provided in a blood-derived sample.
75. Use *ex vivo* of an HCRTR1 protein, or an antigenically active fragment thereof, for the selection or purification of a diagnostic agent for prostate cancer.
76. Use of an HCRTR1 protein, or an antigenically active fragment thereof, for the production of a diagnostic agent for prostate cancer.
77. Use according to item 75 or 76, wherein the diagnostic agent is an affinity ligand capable of selective interaction with the HCRTR1 protein.
78. Use according to any one of items 73-77, wherein the HCRTR1 protein comprises of consists of amino acid sequence SEQ ID NO:7 or 8.
79. Use of an antigenically active fragment according to any one of items 73-78, which fragment is a HCRTR1 protein fragment according to item 72.
80. Affinity ligand capable of selective interaction with the HCRTR1 protein.
81. Affinity ligand according to item 80, wherein the HCRTR1 protein comprises or consists of amino acid sequence SEQ ID NO:7 or 8.
82. Affinity ligand according to item 80 capable of selective interaction with a fragment consisting of amino acid sequence SEQ ID NO:6.
83. Affinity ligand according to any one of items 80-82, which is capable of selective interaction with the HCRTR1 protein in optionally diluted serum or plasma.
84. Affinity ligand according to any one of items 80-83, which is immobilized onto a solid support suitable for a sandwich assay or bound to a bead.
85. Use *ex vivo* of an affinity ligand according to any one of items 80-84 as a diagnostic agent for prostate cancer.
86. Kit comprising:
   a) a first affinity ligand capable of selective interaction with a HCRTR1 protein;
   b) a quantifiable second affinity ligand capable of interaction with the HCRTR1 protein when bound to the first affinity ligand; and optionally
   c) a solid support on which the first affinity ligand is immobilized or which is prepared for immobilization of the first affinity ligand.
87. Kit according to item 86, wherein the quantifiable second affinity ligand is capable of selective interaction with the HCRTR1 protein when bound to the first affinity ligand.
88. Kit according to item 86 or 87, wherein the first affinity ligand interacts with an HCRTR1 amino acid sequence which is different from the HCRTR1 amino acid sequence which the quantifiable second affinity ligand interacts with.
89. Kit according to any one of items 86-88, wherein the quantifiable second affinity ligand is labeled with a quantifiable label.
90. Kit according to any one of items 86-89, further comprising an agent capable of binding the quantifiable second affinity ligand, which agent is labeled with a quantifiable label.
91. Kit according to item 90, wherein the quantifiable second affinity ligand is an antibody and the agent is an antibody capable of binding the Fc region of the quantifiable second affinity ligand.
92. Kit according to any one of items 89-91, wherein the quantifiable label is selected from the group consisting of fluorescent dyes and metals, chromophoric dyes, chemiluminescent compounds and bioluminescent proteins, enzymes, radioisotopes, particles and quantum dots.
93. Kit according to item 92, in which the quantifiable label is an enzyme, further comprising a chromogenic ELISA substrate.
94. Kit according to item 91 or 92, wherein the quantifiable label is an enzyme selected from horseradish peroxidase, alkaline phaosphatase, beta-D-galactosidase and beta-lactamase.
95. Kit according to any one of items 86-94, wherein the first affinity ligand is an antibody.
96. Kit according to any one of items 86-95, wherein the quantifiable second affinity ligand is an antibody.
97. Kit according to any one of items 86-96, wherein the solid support comprises wells in which the first affinity ligand is immobilized or which are prepared for immobilization of the first affinity ligand.

### Brief description of the figures

Figure 1 shows an alignment of the two CNDP1 splice variants as well as the two protein fragments used for antibody production. The highlighted N residues are the two glycosylation sites.
   "CNDP_001" and "CNDP_201" represent the splice variants SEQ ID NO: 3 and 4, respectively. "PrEST1" and "PrEST2" correspond to SEQ ID NO: 1 and 2, respectively.
Figure 2 shows boxplots of differences in protein concentration in serum between two patient groups, A and B, representing a high risk of prostate cancer and a low risk of prostate cancer, respectively. Figure 2A shows results for the anti-CNDP1 antibody and Figure 2B shows results for the anti-HCRTR1 antibody.
Figure 3 shows Western blot validation of the two protein biomarkers CNDP1 and HCRTR1. In the first lane, the marker size is indicated, lane 2, 4 and 6 shows samples from patients with a high risk of prostate cancer, lane 3, 5, and 7 shows samples from patients with a low risk of prostate cancer. Figure 3A shows Western blot for the CNDP1 protein. Figure 3B shows Western blot for the HCRTR1 protein.
Figure 4 shows Western blot analysis of immuno-captured proteins.
   The arrow indicates the unglycosylated CNDP1 protein. In the first lane, the marker bands are indicated, the second lane shows samples from patients with a low risk of prostate cancer, and the third lane shows samples from patients with a high risk of prostate cancer.
Figure 5 shows a boxplot of differences in protein concentration in serum between patients with less aggressive prostate cancer and patients with more aggressive prostate cancer. The level of CNDP1 protein in serum of healthy patients is also shown as control.
Figure 6 shows the results from a sandwich detection assay measuring the levels of the CNDP1 protein. Samples 1 to 3 represent healthy patients and samples 4 to 6 represent patients with aggressive prostate cancer.

### Description of the present disclosure

### Unglycosylated CNDP1 protein

As a first aspect of the present disclosure, there is thus provided a method of determining whether a mammalian subject belongs to a first or a second group of subjects, wherein the risk of having a prostate cancer, such as an aggressive prostate cancer, is higher in the first group than in the second group, comprising the steps of:
a) evaluating an amount of unglycosylated CNDP1 protein in a sample derived from blood, semen or urine from the subject and determining a sample value corresponding to the evaluated amount;
b) comparing the sample value with a predetermined reference value; and if the sample value is lower than or equal to the reference value,
c1) concluding that the subject belongs to the first group; and if the sample value is higher than the reference value,
c2) concluding that the subject belongs to the second group.

The present disclosure is based on the inventors' finding that the unglycosylated form of the protein CNDP1 is less abundant in blood-derived samples from subjects having aggressive prostate cancer than in such samples from healthy subjects or subjects having less aggressive prostate cancer. The inventors have thus concluded that the unglycosylated CNDP1 protein is a biomarker for relatively favorable prostate conditions. In other words, the presence of the unglycosylated CNDP1 protein is indicative of the relatively favorable prostate conditions.

The incidence rate of prostate cancer is high and the outcome of the disease may eventually be death, especially if not treated in time, e.g. before metastasizing. However, some prostate cancer subjects can survive and maintain a good quality of life without treatment. Further, the side-effects of prostate cancer treatment are often severe, including incontinence, impotence and infertility. Also, the treatment in itself can be uncomfortable and/or painful.

Consequently, there is a clinical need for the method of the first aspect, which may be used in detection of prostate cancer, in particular the aggressive forms thereof, at an early stage.

Also, there is a clinical need for discriminating between non-aggressive and aggressive prostate cancer forms. For example, the method of the first aspect may be used for selecting subjects for further diagnosis. Such further diagnosis, or conclusive diagnosis, normally includes prostate tissue biopsies, which should, due to the discomfort and pain involved, be avoided if not necessary. Further, the method according to the first aspect may assist the physician and the subject in the selection of an appropriate treatment (or non-treatment) strategy.

Screening for the unglycosylated CNDP1 protein may thus be an alternative or complement to PSA screening in prostate cancer. Further, an analysis of the level of unglycosylated CNDP1 protein may be performed later in connection with a biopsy or biopsy analysis. Also, the level unglycosylated CNDP1 protein may be considered (e.g. together with the Gleason score) when assessing the risk after the presence of a prostate tumor has been established. In the case when a subject shows high PSA values, but biopsies show no tumor cells, the subject is normally monitored, and the level of unglycosylated CNDP1 protein may be considered during such monitoring (high levels of unglycosylated CNDP1 protein would in such case be indicative of a healthy subject). Further, the inventors believe low levels of unglycosylated CNDP1 detected during treatment follow-up could be indicative of prostate cancer recurrence.

From the reasoning above, the benefits of the further aspects presented below will be evident to the person skilled in the art.

In the methods of the first aspect, it is determined whether a subject belongs to a first or a second group, wherein subjects of the first group generally have a higher risk of having aggressive prostate cancer than subjects of the second group. The division of subjects into the two groups is determined by comparing samples values from the subjects with a reference value. The reference value is thus the determinant for the size of the respective groups; the lower the reference value, the fewer the subjects in the first group and the lower the likelihood that a tested subject belongs to the first group.

The first and the second group may consist exclusively of subjects having the same or similar characteristics as the tested subject. For example, the groups may consist of subjects having the same or similar age, race, nationality, residence, genetic characteristics, medical status (such as PSA level, Gleason score and/or prostate cancer status/stage) and/or medical history (such as prostate cancer history).

A method of the present disclosure may also be based on the degree of glycosylation of CNDP1 protein in a sample derived from blood, semen or urine from the tested subject.

As a configuration of the first aspect, there is thus provided a method of determining whether a mammalian subject belongs to a first or a second group of subjects, wherein the risk of having a prostate cancer, such as an aggressive prostate cancer, is higher in the first group than in the second group, comprising the steps of:
a) quantifying the degree of glycosylation of CNDP1 protein in a sample derived from blood, semen or urine from the subject;
b) comparing the degree of glycosylation of step a) with a predetermined reference degree of glycosylation; and if the degree of glycosylation of step a) is higher than or equal to the reference,
c1) concluding that the subject belongs to the first group; and if the degree of glycosylation of step a) is lower than the reference,
c2) concluding that the subject belongs to the second group.

The degree of glycosylation may for example be obtained by measuring the amount of glycosylated protein in relation to the amount of unglycosylated protein.

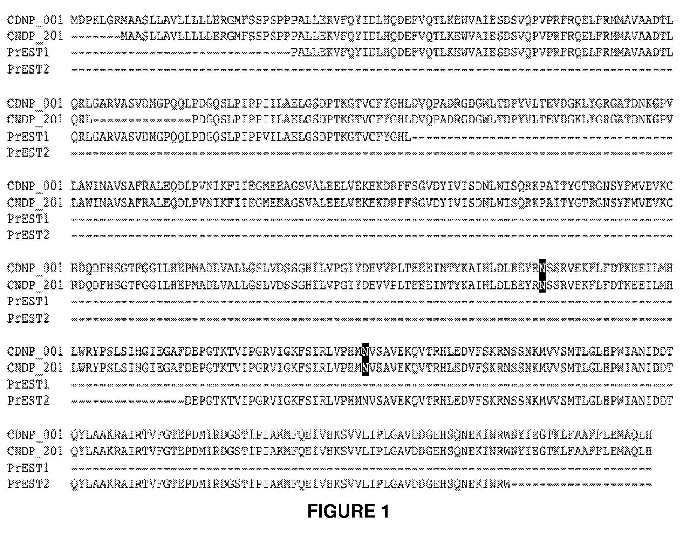

Further, in the determination of the degree of glycosylation, a totally unglycosylated CNDP1 protein may be given more weight than a CNDP1 protein that is only unglycosylated in one of the two positions indicated in figure 1. For example, if the degree of glycosylation is measured using the scale from 0 to 1, protein unglycosylated in both positions may be given the value 0, while protein unglycosylated in one of the positions is given the value 0.5 and protein glycosylated in both positions is given the value 1.

This configuration further applies *mutatis mutandis* to the other method aspects presented below.

As an alternative configuration of the first aspect, there is provided a method for determining a subject's risk of having aggressive prostate cancer, comprising the steps of:
a) evaluating an amount of unglycosylated CNDP1 protein in a sample derived from blood, semen or urine from the subject and determining a sample value corresponding to the evaluated amount; and
b) correlating the sample value of step a) to the risk of having aggressive prostate cancer.

In an embodiment of the alternative configuration, the sample may be an earlier obtained sample.

The correlating of step b) refers to any way of associating data to the obtained sample value so as to determine the risk. With the knowledge of the teachings of the present disclosure, the skilled person may perform step b) without undue burden. For example, the sample value of step a) may be compared to such sample values from reference subjects having prostate cancers of various degrees of aggressiveness and/or healthy reference subjects.

"Risk of having" refers to likelihood of having. The risk may thus be expressed as a percentage (%). Consequently, such a percentage is higher in the first group than in the second group.

The person skilled in the art is capable of selecting a clinically relevant definition of "aggressive prostate cancer". Clinical relevance will thus normally be the main guiding principle when "aggressive prostate cancer" is defined. In addition, the definition may depend on available data and reference samples or values from prostate cancer subjects. For example, if the skilled person had access to the validation cohort of example 8, it would be convenient to select the definition of aggressive prostate cancer used therein.

In embodiments of the present disclosure, "an aggressive prostate cancer" may be:
(i) a metastasizing prostate cancer, such as a prostate cancer of stage T3/T4, N≥1;
(ii) a prostate cancer showing a Gleason sum of 7 or more, such as 8 or more; or
(iii) a prostate cancer from a subject having a PSA level of above 20 ng/ml, such as above 30 or 50 ng/ml.

In some embodiments, the prostate cancer may be classified as aggressive if at least two of the above criteria (i) - (iii) are fulfilled. Further, the prostate cancer may be classified as aggressive if all of the above criteria (i) - (iii) are fulfilled.

The definition of aggressive prostate cancer may also depend on the reference value. The reference value may be selected to single out subjects at risk of having a prostate cancer of certain degree of aggressiveness.

In the context of the present disclosure, the "reference value" refers to a value which can be used for dividing subjects into different categories based on the risk of having aggressive prostate cancer.

With the knowledge of the teachings of the present disclosure, the person skilled in the art may without undue burden select a reference value capable of dividing subjects into two categories according to the methods of the present disclosure. For example, the skilled person may measure the amount of unglycosylated CNDP1 protein in samples from a first group of subjects having an aggressive prostate cancer according to a definition of aggressive prostate cancer. Thereby the skilled person would obtain a first value, such as an average or a median value, corresponding to a prostate cancer of the defined aggressiveness. As a comparison, the skilled person may measure the amount of unglycosylated CNDP1 protein in samples from a second group of healthy subjects or subjects having a less aggressive prostate cancer. Thereby the skilled person would obtain a second value, such as an average or a median value, corresponding to a healthy condition or a less aggressive prostate cancer. A value, which is higher or equal to the first value, but lower than the second value, could then be selected as the reference value.

Likewise, the skilled person can compare values from prostate cancer subjects (having any degree of aggressiveness) with values from healthy patients and thereby derive a reference value for prostate cancer detection.

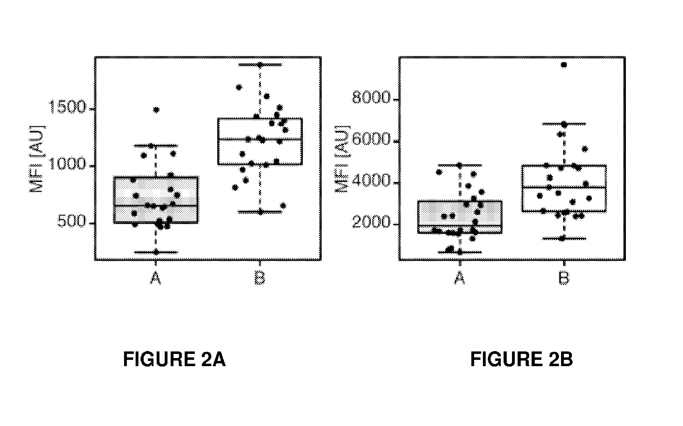

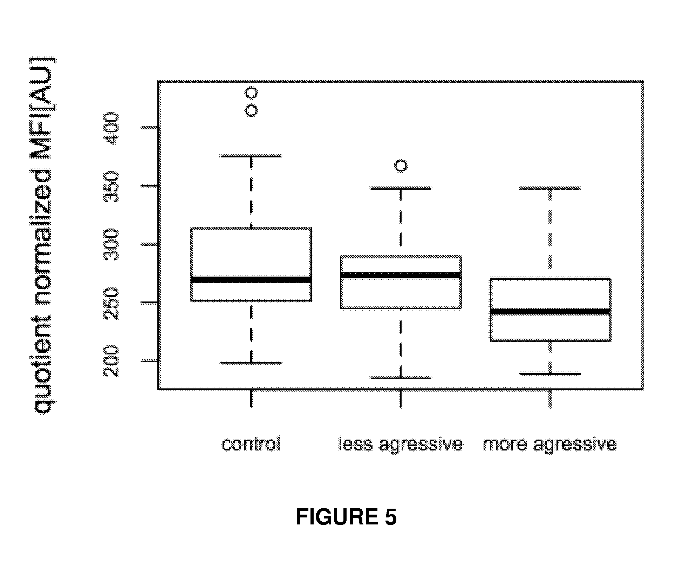

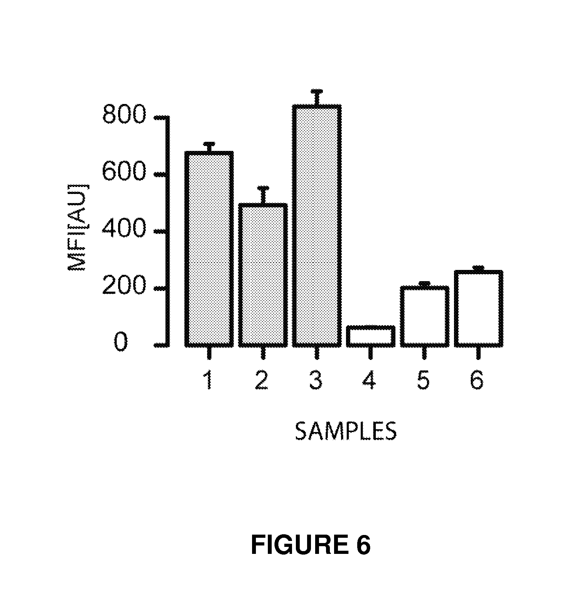

For example, if the protocol and measurement method behind figure 6 is used, a (relative) reference value in the range of 300-480, such as 350-450 can be selected. Further, if the protocol and measurement method behind figure 2b is used, a (relative) reference value of about 3000 can be selected. Also, if the protocol and measurement method behind figure 5 is used, a (relative) reference value of about 260 can be selected.

As explained above, the detection of unglycosylated CNDP1 protein may also be part of the actual detection of prostate cancer. As a second aspect of the present disclosure, there is thus provided a method of detecting a prostate cancer in a mammalian subject, comprising the step of:
a) evaluating an amount of unglycosylated CNDP1 protein in a sample derived from blood, semen or urine from the subject and determining a sample value corresponding to the evaluated amount;
b) comparing the sample value with a predetermined reference value; and if the sample value is lower than or equal to the reference value,
c) examining at least one prostate tissue biopsy from the subject for tumor cells.

In an embodiment, the biopsy is earlier obtained, which means that the step of obtaining the biopsy is not included in the method.

Further, subjects may be selected for more comprehensive examination based on their unglycosylated CNDP1 protein values. As a third aspect of the present disclosure, there is thus provided a method of selecting a mammalian subject for a prostate tissue examination, comprising the steps of:
a) evaluating an amount of unglycosylated CNDP1 protein in a sample derived from blood, semen or urine from the subject and determining a sample value corresponding to the evaluated amount;
b) comparing the sample value with a predetermined reference value; and if the sample value is lower than or equal to the reference value,
c) selecting the subject for the prostate tissue examination.

According to an embodiment of the third aspect, the method further comprises the step:
d) performing the prostate tissue examination.

Further, some embodiments may comprise the step of refraining from the prostate tissue examination if the sample value is higher than the reference value.

The prostate tissue examination may comprise examination of at least one prostate tissue biopsy from the subject. Further, the prostate tissue examination may in some embodiments comprise performing at least one prostate tissue biopsy, such as a transrectal or transperineal biopsy, on the subject. In other embodiments, the performance of the at least one prostate biopsy is not included in the method. The prostate tissue examination may comprise morphological and/or immunohistochemical examination, normally with the purpose of determining if tumor cells are present. In such immunohistochemical examination, tissue slides from the biopsy/biopsies may be stained for basal cells, which are indicative of non-cancerous tissue, e.g. using antibodies for high-molecular cytokeratin or p63 protein, or for a tumor cell marker, such as AMACR. If tumor cells are found, the prostate tissue examination may comprise determining a Gleason score.

"Performing a biopsy" refers to the removal of cells or tissues so they can be viewed under a microscope, e.g. by a pathologist. A "biopsy" refers to the tissue or cells obtained when performing a biopsy. There are mainly two types of biopsy procedures used to diagnose prostate cancer; transrectal biopsy and transperineal biopsy. The performance of the transrectal biopsy is the removal of tissue from the prostate by inserting a thin needle through the rectum and into the prostate. This procedure is usually done using transrectal ultrasound to help guide the needle. The performance of the transperineal biopsy is the removal of tissue from the prostate by inserting a thin needle through the skin between the scrotum and rectum and into the prostate.

It follows from the above discussion that the unglycosylated CNDP1 protein can also be regarded as a prognostic marker. Thus, as a fourth aspect of the present disclosure, there is provided a method for determining whether a mammalian subject belongs to a first or a second group, wherein the prognosis of subjects of the first group is worse than the prognosis of subjects of the second group, comprising the steps of:
a) evaluating an amount of unglycosylated CNDP1 protein in a sample derived from blood, semen or urine from the subject and determining a sample value corresponding to the evaluated amount;
b) comparing the sample value with a predetermined reference value; and if the sample value is lower than or equal to the reference value,
c1) concluding that the subject belongs to the first group; and if the sample value is higher than the reference value,
c2) concluding that the subject belongs to the second group.

In embodiments of the fifth aspect, the subject has a prostate cancer.

In the context of the present disclosure, "prognosis" refers to the prediction of the course or outcome of a disease and its treatment. For example, prognosis may also refer to a determination of chance of survival or recovery from a disease, as well as to a prediction of the expected survival time of a subject. A prognosis may specifically involve establishing the likelihood for survival of a subject during a period of time into the future. A prognosis may further be represented by a single value or a range of values.

The prognosis may thus be a probability of survival. There are several ways to measure "survival". The survival of the present disclosure may for example be overall survival or recurrence free survival. Further, the "survival" may be measured over different periods, such as three, five, ten or 15 years. Accordingly, the survival may be a three-year, five-year, ten-year or 15-year survival.

The finding of the present disclosure may also be used for avoiding overtreatment. As a fifth aspect of the present disclosure, there is thus provided a method for determining whether a mammalian subject is not in need of a prostate cancer treatment regimen, comprising the steps of:
a) evaluating an amount of unglycosylated CNDP1 protein in a sample derived from blood, semen or urine from the subject and determining a sample value corresponding to the evaluated amount;
b) comparing the sample value with a predetermined reference value; and if the sample value is higher than the reference value,
c) concluding that the subject is not in need of the prostate cancer treatment regimen.

As a configuration of the fifth aspect, there is provided a non-treatment strategy method for a mammalian subject, comprising the steps of:
a) evaluating an amount of unglycosylated CNDP1 protein in a sample derived from blood, semen or urine from the subject and determining a sample value corresponding to the evaluated amount;
b) comparing the sample value with a predetermined reference value; and if the sample value is higher than the reference value,
c) refraining from treating the subject with a prostate cancer treatment regimen.

For example, the refraining of step c) of the configuration of the fifth aspect may be a refraining from the treatment regimen during at least one month from the completion of steps a) - b), such as at least three months from the completion of steps a) - b), such as at least six months from the completion of steps a) - b), such as at least nine months from the completion of steps a) - b), such as at least one year from the completion of steps a) - b), such as at least two years from the completion of steps a) - b).

Alternatively, the refraining of step c) may be a refraining from the treatment regimen until the next time the method of the configuration of the fifth aspect is performed or until an aggressive prostate cancer is detected in the subject.

However, subjects having aggressive prostate cancer are normally in need of treatment. As a sixth aspect of the present disclosure, there is thus provided a method of treatment of a mammalian subject having a prostate cancer, comprising the steps of:
a) evaluating an amount of unglycosylated CNDP1 protein in a sample derived from blood, semen or urine from the subject and determining a sample value corresponding to the evaluated amount;
b) comparing the sample value with a predetermined reference value; and if the sample value is lower than or equal to the reference value,
c) treating the subject with a prostate cancer treatment regimen.

The method of treatment may be limited to the decision-making and treatment. Thus, as a configuration of the sixth aspect, there is provided a method of treatment of a mammalian subject having a prostate cancer, comprising the steps of:
α) comparing a sample value corresponding to a level of unglycosylated CNDP1 protein in a sample derived from blood, semen or urine from the subject with a predetermined reference value; and if the sample value is lower than or equal to the reference value,
β) treating the subject with a prostate cancer treatment regimen.

Ways of obtaining a sample value corresponding to a level of unglycosylated CNDP1 protein in a sample are described in the present disclosure.

The level of unglycosylated CNDP1 protein may also be considered when deciding the intensity of a prostate cancer treatment. As a second configuration of the sixth aspect of the present disclosure, there is thus provided a method for determining the level of intensity of a treatment of a mammalian subject having a prostate cancer, comprising the steps of:
a) evaluating an amount of unglycosylated CNDP1 protein in a sample derived from blood, semen or urine from the subject and determining a sample value corresponding to the evaluated amount;
b) comparing the sample value with a predetermined reference value; and if the sample value is lower than or equal to the reference value,
c1) concluding that said subject should be given a treatment of a first intensity; and if the sample value is higher than the reference value,
c2) concluding that said subject should be given a treatment of a second intensity;
wherein the first intensity is higher than the first intensity.

The level of intensity may for example be measured as the average daily or weekly dose of a therapeutic agent given to the subject. A treatment of the first intensity may thus be applied more frequently or in higher individual doses than a treatment of the first second. The treatment of the first intensity may also comprise application of a more aggressive therapy than the treatment of the second intensity. For example, the treatment of the first intensity may be a combination treatment while the treatment of the second intensity is a mono-therapy. Yet another possibility is that the treatment of the first intensity is applied for a longer period than the treatment of the second intensity.

In an embodiment of the third aspect, c1) may thus be concluding that said subject should undergo treatment during a first period and c2) may be concluding that said subject should undergo treatment during a second period, wherein the first period is longer than the second period.

In an embodiment of the third aspect, c1) may thus be concluding that said subject should undergo treatment during a first period and c2) may be concluding that said subject should undergo treatment during a second period, wherein the first period is longer than the second period.

In an alternative or complementary embodiment of the third aspect, c1) may thus be concluding that said subject should undergo first prostate cancer treatment regimen and c2) may be concluding that said subject should undergo second prostate cancer treatment regimen, wherein the first prostate cancer treatment regimen is more aggressive than the second prostate cancer treatment regimen. For a given prostate cancer subject, the person of skill in the art understands, under the circumstances, what to consider a more aggressive and a less aggressive treatment regimen, respectively.

The prostate cancer treatment regimen of the present disclosure may for example be selected from surgery, radiation therapy, hormone therapy, cryosurgery, chemotherapy, immunotherapy, high-intensity focused ultrasound and combinations thereof.

The surgery may for example be retropubic prostatectomy, perineal prostatectomy or laparoscopic prostatectomy.

Radiation therapy is a cancer treatment that uses high-energy x-rays or other types of radiation to kill cancer cells or keep them from growing. There are mainly two types of radiation therapy that are considered in the methods of the present disclosure; external radiation therapy (e.g. 3-D conformal radiation therapy or intensity-modulated) and internal radiation therapy (brachytherapy). External radiation therapy uses a machine outside the body to send radiation towards the cancer. Internal radiation therapy uses a radioactive substance sealed in needles, seeds, wires, catheters or the like that are placed directly into or near the cancer. The way the radiation therapy is given depends on the type and stage of the cancer being treated.

Hormone therapy is a cancer treatment that removes hormones or blocks their action and thereby stops cancer cells from growing. Hormones are substances produced by glands in the body and circulated in the bloodstream. In prostate cancer, male sex hormones can cause prostate cancer to grow. The hormone therapy of the present disclosure may for example be:
application of a luteinizing hormone-releasing hormone agonist, such as leuprolide, goserelin or buserelin (such an agonist can prevent the testicles from producing testosterone);
application of an antiandrogen, such as flutamide or nilutamide (such an antiandrogen can block the action of androgens, i.e. hormones that promote male sex characteristics);
application of a drugs that can prevent the adrenal glands from making androgens, such as ketoconazole or aminoglutethimide;
orchiectomy, which is a surgical procedure to remove one or both testicles, the main source of male hormones, to decrease hormone production; and
application of an estrogen (hormones that promote female sex characteristics) can prevent the testicles from producing testosterone.

Cryosurgery (or cryoablation) is a treatment in which prostate cancer cells are freezed and destroyed. Cryosurgery is usually performed with a special instrument (normally comprising hollow needles/cryoprobes) that contains a cold fluid, such as liquid nitrogen or liquid carbon dioxide.

Chemotherapy is a cancer treatment that uses drugs to stop the growth of cancer cells, either by killing the cells or by stopping them from dividing. The chemotherapy of the present disclosure may for example be selected from Docetaxel, Mitozantrone, Epirubicin, Paclitaxel and Estramustine.

Biological therapy (sometimes referred to as biotherapy, immunotherapy or BRM therapy) is a treatment that uses the patient's immune system to fight cancer. Substances made by the body or made in a laboratory are mainly used to boost, direct, or restore the body's natural defenses against cancer, but can also be used to lessen certain side effects that may be caused by some cancer treatments. Agents used in biological therapy include (therapeutic) monoclonal antibodies known to the skilled person. These agents may have a direct antitumor effect.

High-intensity focused ultrasound (ultrasonography) is a treatment that uses ultrasound (high-energy sound waves) to destroy cancer cells. To treat prostate cancer, an endorectal probe is used to make the sound waves; a procedure in which high-energy sound waves (ultrasound) are bounced off internal tissues or organs and make echoes. The echo patterns may be shown on a screen of an ultrasound machine, forming a picture of body tissues called a sonogram.

The radiation therapy, hormone therapy, chemotherapy, immunotherapy, high-intensity focused ultrasound may be adjuvant or neoadjuvant.

The steps of the above method may be performed by a single person, such as a lab technician, nurse or physician. However, a physician may also perform step c) and optionally step b) himself, while assigning the performance of step a) and optionally step b) to someone else, typically a lab technician or nurse. Also, the physician responsible for the diagnosis and treatment may be one person, normally an urologist, while the physician performing the prostate tissue examination is another person, normally a pathologist. If the above methods involve more than one person, the persons may be situated at different locations and have different employers. For example, the physician may be employed by a hospital while the lab technician is employed by a lab. Samples and information may thus be transferred from one person and location to another within the framework of the methods of the present disclosure.

Regarding step a) of the methods of the present disclosure, an increase in the amount of unglycosylated CNDP1 protein typically results in an increase in the sample value, and not the other way around. However, in some embodiments, the evaluated amount may correspond to any of a predetermined number of discrete sample values. In such embodiments, a first amount and a second, increased, amount may correspond to the same sample value. In any case, an increase in the amount of unglycosylated CNDP1 protein will not result in a decrease in the sample value in the context of the present disclosure.

However inconvenient, but in an equivalent fashion, the evaluated amounts may be inversely related to sample values if the qualification between step b) and c) is inverted. For example, the qualification between step b) and c) is inverted if the phrase "if the sample value is lower than or equal to the reference value" is replaced with "if the sample value is higher than or equal to the reference value".

The methods and uses of the present disclosure, except the methods of treatment, may in some embodiments, unless otherwise stated, be carried out entirely *ex vivo*.

In step a) of the above methods, an amount is evaluated and a sample value corresponding to the amount is determined. Consequently, an exact measurement of the amount of unglycosylated CNDP1 protein is not required for obtaining the sample value. In some embodiments, it may be sufficient to determine if the amount is "high" or "low".

The evaluation and determination of step a) requires some kind of processing and/or manipulation of biological material from the subject. It is not possible to determine the sample value by mere inspection of a blood, semen or urine sample from the subject. Various techniques, of which some are presented below, for such evaluation and determination, are well known to the skilled person. The methods of the present disclosure are therefore not limited to any specific technique or techniques for the performance of step a).

Semen contains fluid from the prostate, and urine is another biological fluid that passes the male genitals. Therefore, the sample of the present disclosure may not only be derived from blood, but also from semen or urine. However, since the finding of the present disclosure is based on blood-derived samples, the sample of the present disclosure is preferably derived from blood.

The sample derived from blood may for example be a serum or plasma sample. The sample may be diluted (e.g. in buffer) to facilitate handling and detection of the unglycosylated CNDP1 protein.

In the context of the present disclosure, an "unglycosylated CNDP1 protein" refers to a CNDP1 protein which is unglycosylated in at least one of the two positions indicated in figure 1.

The inventors believe that the level of glycosylated CNDP1 may increase as the level of unglycosylated CNDP1 decreases, and it is therefore possible that measurements of the level of the glycosylated form indirectly yield the level of the unglycosylated form.

In embodiments of the present disclosure, the unglycosylated CNDP1 protein is unglycosylated in both the positions indicated in figure 1.

The unglycosylated form of CNDP1 may for example be selectively detected by an affinity ligand, such as an antibody. Such an affinity ligand may for example bind to an epitope or region overlapping one or both of the glycosylation sites seen in figure 1 or to an epitope or region which is sterically hindered when the protein is glycosylated. In some embodiments of the methods of the present disclosure, an affinity ligand capable of distinguishing between the glycosylated and unglycosylated form of CNDP1 protein is thus employed.

Further, the skilled person should recognize that the usefulness of the above methods or other aspects of the present disclosure is not limited to the quantification of any particular variant of the unglycosylated CNDP1 protein present in the subject in question, as long as the protein is encoded by the relevant gene and presents the relevant pattern of expression.

As a non-limiting example, the unglycosylated CNDP1 protein of the present disclosure may comprise a sequence selected from:
i) SEQ ID NO:3;
ii) SEQ ID NO:4; and
iii) a sequence which is at least 85 % identical to SEQ ID NO:3 or 4.

In some embodiments, sequence ii) above is at least 90 % identical, at least 91 % identical, at least 92 % identical, at least 93 % identical, at least 94 % identical, at least 95 % identical, at least 96 % identical, at least 97 % identical, at least 98 % identical or at least 99 % identical to SEQ ID NO:3 or 4.

The term "% identical", as used in the context of the present disclosure, is calculated as follows. The query sequence is aligned to the target sequence using the CLUSTAL W algorithm (Thompson, J.D., Higgins, D.G. and Gibson, T.J., Nucleic Acids Research, 22: 4673-4680 (1994)). The amino acid residues at each position are compared, and the percentage of positions in the query sequence that have identical correspondences in the target sequence is reported as % identical. Also, the target sequence determines the number of positions that are compared. Consequently, in the context of the present disclosure, a query sequence that is shorter than the target sequence can never be 100 % identical to the target sequence. For example, a query sequence of 85 amino acids may at the most be 85 % identical to a target sequence of 100 amino acids.

In some embodiments, step a) of the methods of the above aspects may comprise obtaining blood from the subject and preparing serum or plasma from the obtained blood to facilitate the evaluation of step a). Step a) may further comprise diluting the serum or plasma, e.g. in one or more buffers, at least 10 times, such as at least 50 times. Also, step a) may comprise heating the sample to a temperature in the range of 37-70 °C, such as 45-65 °C, such as 50-60 °C, before evaluating the amount of the unglycosylated CNDP1 protein. The heating may for example be performed for 5 - 120 minutes, such as 10 - 45 minutes. Such heating has been shown by the inventors to increase the immunodetectability of proteins in blood-derived samples.

In embodiments of the above methods, step a) may comprise contacting the sample with an affinity ligand that is, under the conditions of the contact, capable of selective interaction with the unglycosylated CNDP1 protein.

An affinity ligand may be capable of selective interaction with a target in a first set-up, but not in a second. For example, an antibody may be capable of selective interaction with the target in a sandwich assay (see below), in which an optionally diluted semen, urine, blood, serum or plasma sample containing the target is contacted with the antibody immobilized on a solid phase, while the same antibody is not capable of selective interaction with the target in a western blot experiment.

In the context of the present disclosure, "selective" interaction of e.g., an affinity ligand with its target or antigen means that the interaction is such that a distinction between selective and non-selective interaction becomes meaningful. The interaction between two proteins is sometimes measured by the dissociation constant. The dissociation constant describes the strength of binding (or affinity) between two molecules. Typically the dissociation constant between an antibody and its antigen is from 10⁻⁷ to 10⁻¹¹ M. However, high selectivity does not necessarily require high affinity. Molecules with low affinity (in the molar range) for its counterpart have been shown to be as selective as molecules with much higher affinity. In the case of the present disclosure, a selective interaction refers to the extent to which a particular method can be used to determine the presence and/or amount of a specific protein, the target protein, under given conditions in the presence of other proteins in a fluid sample of a naturally occurring or processed biological fluid (e.g. serum or plasma). In other words, selectivity is the capacity to distinguish between related proteins. "Specific" and "selective" are sometimes used interchangeably in the present description. For example, the specificity or selectivity of an antibody may be determined as in Examples, Section 2, below. Specificity and selectivity determinations are also described in Nilsson P et al. (2005) Proteomics 5:4327-4337.

Step a) may further comprise quantification of the interaction between the affinity ligand and the unglycosylated CNDP1 protein to obtain the evaluated amount.

As indicated above, step a) may for example comprise a sandwich assay.

In the context of the present disclosure, a "sandwich assay" refers to an assay comprising:
contacting the sample with a first affinity ligand (capture affinity ligand) immobilized to a solid support such, which first affinity ligand is capable of selective interaction with unglycosylated CNDP1 protein under the conditions of the contact;
removing unbound affinity ligand (e.g. washing the solid support); and
contacting the solid support with a second affinity ligand (detection affinity ligand) capable of interaction with unglycosylated CNDP1 protein bound to the first affinity ligand, which second affinity ligand is directly or indirectly detectable.

Unbound affinity ligand may be removed again after the contact with the second affinity ligand (e.g. another washing) and the amount of bound second affinity ligand may then be detected and/or quantified.

The second affinity ligand may thus comprise a detectable label, such as a quantifiable label. Alternatively, the second affinity ligand may be detectable by an agent comprising the detectable label, such as the quantifiable label. For example, the second affinity ligand may be a goat antibody, and the agent may be a labeled anti-goat antibody. Several labels enabling detection and/or quantification are discussed below.

Since the antigen is recognized twice with intermediate removal of unspecific binding, the sandwich assay facilitates sensitive and quantifiable detection of unglycosylated CNDP1 protein in a sample derived from blood, semen or urine.

The sandwich assay of the present disclosure may for example be an ELISA sandwich assay, which is a technique for quantifying the amount of antigen in a sample, such as a blood-derived sample.

The skilled person may, without undue burden, implement a sandwich or ELISA assay in the above methods using the teachings of the present disclosure (ELISA is also further discussed below).

Further sandwich assay embodiments of the above methods are evident from the discussion about sandwich assays in connection with the kit aspect below.

It is regarded as within the capabilities of those of ordinary skill in the art to select or manufacture the proper affinity ligand and to select the proper format and conditions for detection and/or quantification. Nevertheless, examples of affinity ligands that may prove useful, as well as examples of formats and conditions for detection and/or quantification, are given below for the sake of illustration.

Thus, in embodiments of the present disclosure, the affinity ligand may be selected from the group consisting of antibodies, fragments thereof and derivatives thereof, i.e., affinity ligands based on an immunoglobulin scaffold. The antibodies and the fragments or derivatives thereof may be isolated. Antibodies comprise monoclonal and polyclonal antibodies of any origin, including murine, rabbit, human and other antibodies, as well as chimeric antibodies comprising sequences from different species, such as partly humanized antibodies, e.g., partly humanized mouse antibodies. Polyclonal antibodies are produced by immunization of animals with the antigen of choice. The polyclonal antibodies may be mono-specific. Monoclonal antibodies of defined specificity can be produced using the hybridoma technology developed by Köhler and Milstein (Köhler G and Milstein C (1976) Eur. J. Immunol. 6:511-519). The antibody fragments and derivatives of the present disclosure are capable of selective interaction with the same antigen (e.g. unglycosylated CNDP1) as the antibody they are fragments or derivatives of. Antibody fragments and derivatives comprise Fab fragments, consisting of the first constant domain of the heavy chain (CH1), the constant domain of the light chain (CL), the variable domain of the heavy chain (VH) and the variable domain of the light chain (VL) of an intact immunoglobulin protein; Fv fragments, consisting of the two variable antibody domains VH and VL (Skerra A and Plückthun A (1988) Science 240:1038-1041); single chain Fv fragments (scFv), consisting of the two VH and VL domains linked together by a flexible peptide linker (Bird RE and Walker BW (1991) Trends Biotechnol. 9:132-137); Bence Jones dimers (Stevens FJ et al. (1991) Biochemistry 30:6803-6805); camelid heavy-chain dimers (Hamers-Casterman C et al. (1993) Nature 363:446-448) and single variable domains (Cai X and Garen A (1996) Proc. Natl. Acad. Sci. U.S.A. 93:6280-6285; Masat L et al. (1994) Proc. Natl. Acad. Sci. U.S.A. 91:893-896), and single domain scaffolds like e.g., the New Antigen Receptor (NAR) from the nurse shark (Dooley H et al. (2003) Mol. Immunol. 40:25-33) and minibodies based on a variable heavy domain (Skerra A and Plückthun A (1988) Science 240:1038-1041).

In the context of the present disclosure, a "mono-specific antibody" is a population of polyclonal antibodies which has been affinity purified on its own antigen, thereby separating such mono-specific antibodies from other antiserum proteins and non-specific antibodies. This affinity purification results in antibodies that bind selectively to its antigen. In the case of the present disclosure, the polyclonal antisera are purified by a two-step immunoaffinity based protocol to obtain mono-specific antibodies selective for the target protein. Antibodies directed against generic affinity tags of antigen fragments are removed in a primary depletion step, using the immobilized tag protein as the capturing agent. Following the first depletion step, the serum is loaded on a second affinity column with the antigen as capturing agent, in order to enrich for antibodies specific for the antigen (see also Nilsson P et al. (2005) Proteomics 5:4327-4337).

The CNDP1 fragments SEQ ID NO:1 and 2 were designed to lack transmembrane regions to ensure efficient expression in *E. coli*, and to lack any signal peptide, since those are cleaved off in the mature protein. SEQ ID NO:1 and 2 were thus designed for immunizations. In addition, the protein fragments were designed to consist of a unique sequence with low homology with other human proteins, to minimize cross reactivity of generated affinity reagents, and to be of a suitable size to allow the formation of conformational epitopes and still allow efficient cloning and expression in bacterial systems. Accordingly, in the cases wherein the affinity ligand is an antibody or fragment o derivative thereof, the affinity ligand may be obtainable by a process comprising a step of immunizing an animal with a peptide whose amino acid sequence consists of sequence SEQ ID NO:1 or 2. For example, the immunization process may comprise primary immunization with the protein in Freund's complete adjuvant. Also, the immunization process may further comprise boosting at least two times, in intervals of 2-6 weeks, with the protein in Freund's incomplete adjuvant. Processes for the production of antibodies or fragments or derivatives thereof against a given target are known in the art.

Polyclonal and monoclonal antibodies, as well as their fragments and derivatives, represent the traditional choice of affinity ligands in applications requiring selective biomolecular recognition, such as in the detection and/or quantification of unglycosylated CNDP1 protein according to the method aspects above. However, those of skill in the art know that, due to the increasing demand of high throughput generation of selective binding ligands and low cost production systems, new biomolecular diversity technologies have been developed during the last decade. This has enabled a generation of novel types of affinity ligands of both immunoglobulin as well as non-immunoglobulin origin that have proven equally useful as binding ligands in biomolecular recognition applications and can be used instead of, or together with, immunoglobulins.

The biomolecular diversity needed for selection of affinity ligands may be generated by combinatorial engineering of one of a plurality of possible scaffold molecules, and specific/selective affinity ligands are then selected using a suitable selection platform. The scaffold molecule may be of immunoglobulin protein origin (Bradbury AR and Marks JD (2004) J. Immunol. Meths. 290:29-49), of non-immunoglobulin protein origin (Nygren PÅ and Skerra A (2004) J. Immunol. Meths. 290:3-28), or of an oligonucleotide origin (Gold L et al. (1995) Annu. Rev. Biochem. 64:763-797).

A large number of non-immunoglobulin protein scaffolds have been used as supporting structures in development of novel binding proteins. Non-limiting examples of such structures, useful for generating affinity ligands against unglycosylated CNDP1 protein for use according to the present disclosure, are staphylococcal protein A and domains thereof and derivatives of these domains, such as protein Z (Nord K et al. (1997) Nat. Biotechnol. 15:772-777); lipocalins (Beste G et al. (1999) Proc. Natl. Acad. Sci. U.S.A. 96:1898-1903); ankyrin repeat domains (Binz HK et al. (2003) J. Mol. Biol. 332:489-503); cellulose binding domains (CBD) (Smith GP et al. (1998) J. Mol. Biol. 277:317-332; Lehtiö J et al. (2000) Proteins 41:316-322); y crystallines (Fiedler U and Rudolph R, WO01/04144); green fluorescent protein (GFP) (Peelle B et al. (2001) Chem. Biol. 8:521-534); human cytotoxic T lymphocyte-associated antigen 4 (CTLA-4) (Hufton SE et al. (2000) FEBS Lett. 475:225-231; Irving RA et al. (2001) J. Immunol. Meth. 248:31-45); protease inhibitors, such as Knottin proteins (Wentzel A et al. (2001) J. Bacteriol. 183:7273-7284; Baggio R et al. (2002) J. Mol. Recognit. 15:126-134) and Kunitz domains (Roberts BL et al. (1992) Gene 121:9-15; Dennis MS and Lazarus RA (1994) J. Biol. Chem. 269:22137-22144); PDZ domains (Schneider S et al. (1999) Nat. Biotechnol. 17:170-175); peptide aptamers, such as thioredoxin (Lu Z et al. (1995) Biotechnology 13:366-372; Klevenz B et al. (2002) Cell. Mol. Life Sci. 59:1993-1998); staphylococcal nuclease (Norman TC et al. (1999) Science 285:591-595); tendamistats (McConell SJ and Hoess RH (1995) J. Mol. Biol. 250:460-479; Li R et al. (2003) Protein Eng. 16:65-72); trinectins based on the fibronectin type III domain (Koide A et al. (1998) J. Mol. Biol. 284:1141-1151; Xu L et al. (2002) Chem. Biol. 9:933-942); and zinc fingers (Bianchi E et al. (1995) J. Mol. Biol. 247:154-160; Klug A (1999) J. Mol. Biol. 293:215-218; Segal DJ et al. (2003) Biochemistry 42:2137-2148).

The above-mentioned examples of non-immunoglobulin protein scaffolds include scaffold proteins presenting a single randomized loop used for the generation of novel binding specificities, protein scaffolds with a rigid secondary structure where side chains protruding from the protein surface are randomized for the generation of novel binding specificities, and scaffolds exhibiting a non-contiguous hyper-variable loop region used for the generation of novel binding specificities.

In addition to non-immunoglobulin proteins, oligonucleotides may also be used as affinity ligands. Single stranded nucleic acids, called aptamers or decoys, fold into well-defined three-dimensional structures and bind to their target with high affinity and specificity. (Ellington AD and Szostak JW (1990) Nature 346:818-822; Brody EN and Gold L (2000) J. Biotechnol. 74:5-13; Mayer G and Jenne A (2004) BioDrugs 18:351-359). The oligonucleotide ligands can be either RNA or DNA and can bind to a wide range of target molecule classes.

For selection of the desired affinity ligand from a pool of variants of any of the scaffold structures mentioned above, a number of selection platforms are available for the isolation of a specific novel ligand against a target protein of choice. Selection platforms include, but are not limited to, phage display (Smith GP (1985) Science 228:1315-1317), ribosome display (Hanes J and Plückthun A (1997) Proc. Natl. Acad. Sci. U.S.A. 94:4937-4942), yeast two-hybrid system (Fields S and Song O (1989) Nature 340:245-246), yeast display (Gai SA and Wittrup KD (2007) Curr Opin Struct Biol 17:467-473), mRNA display (Roberts RW and Szostak JW (1997) Proc. Natl. Acad. Sci. U.S.A. 94:12297-12302), bacterial display (Daugherty PS (2007) Curr Opin Struct Biol 17:474-480, Kronqvist N et al. (2008) Protein Eng Des Sel 1-9, Harvey BR et al. (2004) PNAS 101 (25):913-9198), microbead display (Nord O et al. (2003) J Biotechnol 106:1-13, WO01/05808), SELEX (System Evolution of Ligands by Exponential Enrichment) (Tuerk C and Gold L (1990) Science 249:505-510) and protein fragment complementation assays (PCA) (Remy I and Michnick SW (1999) Proc. Natl. Acad. Sci. U.S.A. 96:5394-5399).

Thus, in embodiments of the present disclosure, the affinity ligand may be a non-immunoglobulin affinity ligand derived from any of the protein scaffolds listed above, or an oligonucleotide molecule.

There are two splice variants of the CNDP1 protein; SEQ ID NO:3 and 4 (see figure 1). The affinity ligand of the present disclosure may thus be capable of selective interaction with a peptide consisting of amino acid sequence SEQ ID NO:3 and/or with a peptide consisting of amino acid sequence SEQ ID NO:4. A single affinity ligand may thus be capable of recognizing both splice variants, which may be beneficial.

As mentioned above, the CNDP1 fragments were designed to consist of a unique sequence with low homology with other human proteins and to minimize cross reactivity of generated affinity reagents. Consequently, in embodiments of the present disclosure, the affinity ligand may be capable of selective interaction with a polypeptide consisting of amino acid sequence SEQ ID NO:1 or 2.

A benefit of the fragment SEQ ID NO:2 is that its complete amino acid sequence is common to both splice variants of CNDP1.

The detection and/or quantification of the affinity ligand capable of selective interaction with the unglycosylated CNDP1 protein may be accomplished in any way known to the skilled person for detection and/or quantification of binding reagents in assays based on biological interactions. Accordingly, any affinity ligand described above may be used to quantitatively and/or qualitatively detect the presence of the unglycosylated CNDP1 protein. These "primary" affinity ligands may be labeled themselves with various markers or may in turn be detected by secondary, labeled affinity ligands to allow detection, visualization and/or quantification. This can be accomplished using any one or more of a multitude of labels, which can be conjugated to the affinity ligand capable of interaction with unglycosylated CNDP1 protein or to any secondary affinity ligand, using any one or more of a multitude of techniques known to the skilled person, and not as such involving any undue experimentation.

Non-limiting examples of labels that can be conjugated to primary and/or secondary affinity ligands include fluorescent dyes or metals (e.g., fluorescein, rhodamine, phycoerythrin, fluorescamine), chromophoric dyes (e.g., rhodopsin), chemiluminescent compounds (e.g., luminal, imidazole) and bioluminescent proteins (e.g., luciferin, luciferase), haptens (e.g., biotin). A variety of other useful fluorescers and chromophores are described in Stryer L (1968) Science 162:526-533 and Brand L and Gohlke JR (1972) Annu. Rev. Biochem. 41:843-868. Affinity ligands can also be labeled with enzymes (e.g., horseradish peroxidase, alkaline phosphatase, beta-lactamase), radioisotopes (e.g., ³H, ¹⁴C, ³²P, ³⁵S or ¹²⁵I) and particles (e.g., gold). In the context of the present disclosure, "particles" refer to particles, such as metal particles, suitable for labeling of molecules. Further, the affinity ligands may also be labeled with fluorescent semiconductor nanocrystals (quantum dots). Quantum dots have superior quantum yield and are more photostable compared to organic fluorophores and are therefore more easily detected (Chan et al. (2002) Curr Opi Biotech. 13: 40-46). The different types of labels can be conjugated to an affinity ligand using various chemistries, e.g., the amine reaction or the thiol reaction. However, other reactive groups than amines and thiols can be used, e.g., aldehydes, carboxylic acids and glutamine.

The method aspects above may be put to use in any of several known formats and set-ups, of which a non-limiting selection is discussed below.

The method of visualization of labels on the affinity ligand may include, but is not restricted to, fluorometric, luminometric and/or enzymatic techniques. Fluorescence is detected and/or quantified by exposing fluorescent labels to light of a specific wavelength and thereafter detecting and/or quantifying the emitted light in a specific wavelength region. The presence of a luminescently tagged affinity ligand may be detected and/or quantified by luminescence developed during a chemical reaction. Detection of an enzymatic reaction is due to a color shift in the sample arising from a chemical reaction. Those of skill in the art are aware that a variety of different protocols can be modified in order for proper detection and/or quantification.

One available method for detection and/or quantification of the unglycosylated CNDP1 protein is by linking the affinity ligand to an enzyme that can then later be detected and/or quantified in an enzyme immunoassay (such as an EIA or ELISA). Such techniques are well established, and their realization does not present any undue difficulties to the skilled person. In such methods, the biological sample is brought into contact with a solid material or with a solid material conjugated to an affinity ligand against the unglycosylated CNDP1 protein. The affinity ligand-antigen complex is then indirectly or directly detected with an enzymatically labeled secondary affinity ligand. Following this, an appropriate substrate is brought to react in appropriate buffers with the enzymatic label to produce a chemical moiety, which for example is detected and/or quantified using a spectrophotometer, fluorometer, luminometer or by visual means.

As stated above, primary and any secondary affinity ligands can be labeled with radioisotopes to enable detection and/or quantification. Non-limiting examples of appropriate radiolabels in the present disclosure are ³H, ¹⁴C, ³²P, ³⁵S or ¹²⁵I. The specific activity of the labeled affinity ligand is dependent upon the half-life of the radiolabel, isotopic purity, and how the label has been incorporated into the affinity ligand. Affinity ligands are preferably labeled using well-known techniques (Wensel TG and Meares CF (1983) in: Radioimmunoimaging and Radioimmunotherapy (Burchiel SW and Rhodes BA eds.) Elsevier, New York, pp 185-196). A thus radiolabeled affinity ligand can be used to visualize the unglycosylated CNDP1 protein by detection of radioactivity. Radionuclear scanning with e.g., gamma camera, magnetic resonance spectroscopy or emission tomography function for detection *in vivo* and *in vitro,* while gamma/beta counters, scintillation counters and radiographies are also used *in vitro.*

Following the findings presented above, the inventors have realized several uses for the unglycosylated CNDP1 protein and fragments thereof.

Thus, as a seventh aspect of the present disclosure, there is provided a CNDP1 protein fragment which consists of 150 amino acids or less, such as 135 amino acids or less, and comprises or consists of amino acid sequence SEQ ID NO:1 or 2. SEQ ID NO:1 and 2 are 102 and 131 amino acids long, respectively. The fragment is preferably unglycosylated. Possible uses of such a fragment are discussed below.

As an eighth aspect of the present disclosure, there is provided a use *ex vivo* of an unglycosylated CNDP1 protein as a diagnostic marker for prostate cancer. The prostate cancer of the eighth aspect may for example be aggressive prostate cancer. Further, the unglycosylated CNDP1 protein of the eighth aspect may for example be provided in a blood-derived sample.

In the context of the present disclosure, a "diagnostic marker" refers to a biological entity which presence or absence correlates with a medical condition. The marker may thus be a biomarker, such as a human protein. In the present case, the presence of unglycosylated CNDP1 protein is a marker of a healthy condition or an unaggressive prostate cancer, while the absence of unglycosylated CNDP1 protein is a marker of a prostate cancer, such as aggressive prostate cancer.

As an ninth aspect of the present disclosure, there is provided a use *ex vivo* of an unglycosylated CNDP1, or an antigenically active fragment thereof, for the selection or purification of a diagnostic agent for prostate cancer, such as aggressive prostate cancer.

As a tenth aspect of the present disclosure, there is provided a use of an unglycosylated CNDP1 protein, or an antigenically active fragment thereof, for the production of a diagnostic agent for prostate cancer, such as aggressive prostate cancer. The ninth aspect may be *in vivo,* e.g. comprise the immunization of an animal (see below).

In the context of the present disclosure, "diagnostic agent" refers to an agent having at least one property being valuable in an establishment of a diagnosis. For example, the diagnostic agent may be capable of selective interaction with the diagnostic marker. In embodiments of the present disclosure, the diagnostic agent may be an affinity ligand capable of selective interaction with the unglycosylated CNDP1 protein. Examples of such affinity ligands are discussed above in connection with the method aspects.

The antigenically active fragment of aspects nine and ten may be the fragment of aspect seven.

Guided by the teachings of the present disclosure, the person skilled in the art understands how to use the unglycosylated CNDP1 protein or fragment in the production, selection or purification of the diagnostic agent. For example, the use may comprise affinity purification on a solid support onto which the unglycosylated CNDP1 protein or fragment has been immobilized. The solid support may for example be arranged in a column. Further, the use may comprise selection of affinity ligands having specificity for the unglycosylated CNDP1 protein or fragment using a solid support onto which the polypeptide has been immobilized. Such solid support may be well plates (such as 96 well plates), magnetic beads, agarose beads or sepharose beads. Further, the use may comprise analysis of affinity ligands on a soluble matrix, for example using a dextran matrix, or use in a surface plasmon resonance instrument, such as a Biacore™ instrument, wherein the analysis may for example comprise monitoring the affinity for the immobilized unglycosylated CNDP1 protein or fragment of a number of potential affinity ligands.

Also, for the production of the diagnostic agent, the unglycosylated CNDP1 protein or the antigenically active fragment thereof may be used in an immunization of an animal, such as a rabbit or mouse.

Such use may be involved in a method comprising the steps:
i) immunizing an animal using the unglycosylated CNDP1 protein or the antigenically active fragment thereof as the antigen;
ii) obtaining serum comprising the diagnostic agent from the immunized animal; and, optionally,
iii) isolating the diagnostic agent from the serum.

Alternatively the steps following the first step may be:
ii') obtaining cells from the immunized animal, which cells comprise DNA encoding the diagnostic agent,
iii') fusing the cells with myeloma cells to obtain at least one clone, and
iv') obtaining the diagnostic agent expressed by the clone.

In the context of the present disclosure, an "antigenically active fragment" refers of a fragment of sufficient size to generate, by means of an immunization, and antibody capable of selective interaction with the antigenically active fragment. Such a fragment may for example have a length of at least 10 or 15 amino acids residues.

As an eleventh aspect of the present disclosure, there is provided an affinity ligand capable of selective interaction with unglycosylated CNDP1 protein. Different embodiments of such an affinity ligand are discussed above in connection with the method aspects.

The affinity ligand of the eleventh aspect may for example be capable of selective interaction with the unglycosylated form of CNDP1 in optionally diluted serum or plasma. Such an affinity ligand is particularly useful when the amount of unglycosylated CNDP1 protein in blood-derived samples is detected and/or quantified.

Also, the affinity ligand may be arranged for an assay in which unglycosylated CNDP1 protein in samples derived from blood, semen or urine is detected and/or quantified. In embodiments of the eleventh aspect, the affinity ligand may thus be immobilized onto a solid support suitable for a sandwich assay, such as a microwell plate or a bead. Different solid supports are discussed above in connection with the method aspects and below in connection with kit aspect.

As a twelfth aspect of the present disclosure, there is provided a use of an affinity ligand according to the eleventh aspect as a diagnostic agent for prostate cancer, such as aggressive prostate cancer. Consequently, the affinity ligand may be used in the diagnosis of prostate cancer. For example, the affinity ligand may be used in prostate cancer screening. The use may for example be performed *ex vivo,* e.g., involving the determination of the amount of unglycosylated CNDP1 protein in samples derived from blood, semen or urine earlier obtained from a subject.

Components for carrying out the above methods may advantageously be comprised in a kit.

As a thirteenth aspect of the present disclosure, there is thus provided a kit comprising:
a) a first affinity ligand capable of selective interaction with unglycosylated CNDP1 protein;
b) a quantifiable second affinity ligand capable of interaction with unglycosylated CNDP1 protein when bound to the first affinity ligand; and optionally
c) a solid support on which the first affinity ligand is immobilized or which is prepared for immobilization of the first affinity ligand.

In embodiments, the first and second affinity ligand may independently be any one of the affinity ligands described above in connection with the method aspects.

The first affinity ligand is preferably capable of selective interaction with unglycosylated CNDP1 protein in optionally diluted serum or plasma.

Further, the quantifiable second affinity ligand is preferably capable of selective interaction with the unglycosylated CNDP1 protein when bound to the first affinity ligand. However, this is not necessary; only one of the affinity ligands of the kit has to be capable of selective interaction with unglycosylated CNDP1 protein.

Various components of the kit may be selected and specified as described above in connection with the method aspects of the present disclosure. The kit may also contain various auxiliary substances other than affinity ligands, to enable the kit to be used easily and efficiently. The kit may thus further comprise auxiliary substances selected from sample diluents, wash buffers, standard solutions containing unglycosylated CNDP1 protein and substrate for measuring enzyme activity in cases where an enzyme is used as a label.

The standard solution(s) may be used for making a standard curve. The kit may thus comprise a standard solution of one single concentration (to be diluted to different concentrations by the user) or two or more standard solutions of different concentrations.

The kit may also contain a reference sample comprising a known amount of unglycosylated CNDP1 protein. The amount of unglycosylated CNDP1 protein in the reference sample may correspond to a reference value employed in a method according to one of the above aspects. The reference sample may for example comprise plasma or serum.

In some embodiments, the first affinity ligand interacts with a CNDP1 amino acid sequence which is different from the CNDP1 amino acid sequence which the quantifiable second affinity ligand interacts with. An assay in which recognition of two separate epitopes or epitope regions is necessary for generating a signal increases the specificity by reducing the number of false positives.

For example, the first affinity ligand may be capable of selective interaction with a peptide whose amino acid sequence consist of one of SEQ ID NO:1 or SEQ ID NO:2. Further, the quantifiable second affinity ligand may be capable of selective interaction with a peptide whose amino acid sequence consist of the other one of SEQ ID NO:1 or SEQ ID NO:2.

To facilitate the quantification, the quantifiable second affinity ligand may be labeled with a quantifiable label. Alternatively, the kit may further comprise an agent capable of binding the quantifiable second affinity ligand, which agent is labeled with a quantifiable label. Using such an agent may be beneficial since it may be common to different quantifiable second affinity ligands recognizing different antigens.

In some embodiments, the quantifiable second affinity ligand may be an antibody and the agent an antibody capable of binding the Fc region of the quantifiable second affinity ligand. For example, the quantifiable second affinity ligand may be a goat antibody and the agent a labeled anti-goat antibody. Similarly, the quantifiable second affinity ligand may be a mouse antibody and the agent a labeled anti-mouse antibody.

The quantifiable label may for example be selected from the group consisting of fluorescent dyes and metals, chromophoric dyes, chemiluminescent compounds and bioluminescent proteins, enzymes, radioisotopes, particles and quantum dots. Such labels are further discussed above in connection with the method aspects.

The enzyme, which normally implies that the kit is an ELISA kit, may be selected from horseradish peroxidase, alkaline phaosphatase, beta-D-galactosidase and beta-lactamase.

Further, when the quantifiable label is an enzyme, the kit may further comprise a chromogenic ELISA substrate.

The solid support may for example comprise wells in which the first affinity ligand is immobilized or which are prepared for immobilization of the first affinity ligand. Such a solid support may be adapted for a sandwich assay, such as an ELISA sandwich assay. Consequently, the first affinity ligand may be immobilized on a (microwell) plate adapted for an ELISA plate reader.

Alternatively, the solid support may be beads, such as magnetic beads, agarose beads or sepharose beads, to which the first affinity ligand is coupled. Such beads facilitate a miniaturized protocol requiring only small amounts of sample and affinity ligand (see also the examples). Luminex instrumentation, which is commercially available and well-known to the skilled person, may be employed for such a miniaturized protocol. Examples of commercially available beads are Dynabeads and Microsperes from Luminex-Corp.

The kit may also comprise instructions for the quantification of unglycosylated CNDP1 protein in samples derived from blood, urine or semen.

### HCRTR1 protein

As a fourteenth aspect of the present disclosure, there is provided a method of determining whether a mammalian subject belongs to a first or a second group of subjects, wherein the risk of having a prostate cancer is higher in the first group than in the second group, comprising the steps of:
a) evaluating an amount of HCRTR1 protein in a sample derived from blood, semen or urine from the subject and determining a sample value corresponding to the evaluated amount;
b) comparing the sample value with a predetermined reference value; and if the sample value is lower than or equal to the reference value,
c1) concluding that the subject belongs to the first group; and if the sample value is higher than the reference value,
c2) concluding that the subject belongs to the second group.

Above items 54-97, which are related to the HCRTR1 protein, represent further aspects and embodiments of the present disclosure.

HCRTR1-related aspects of the present disclosure are based on the inventors' finding that relatively low amounts of the HCRTR1 protein in blood-derived samples are associated with prostate cancer characteristics (see the examples). The benefits of such a biomarker are evident from the discussion about unglycosylated CNDP1 protein in connection with the first aspect above. Various features of the HCRTR1-related aspects are also defined and explained above in connection with the aspects related to the unglycosylated CNDP1 protein.

The skilled person should recognize that the usefulness of the HCRTR1-related aspects is not limited to the quantification of any particular variant of the HCRTR1 protein present in the subject in question, as long as the protein is encoded by the relevant gene and presents the relevant pattern of expression.

As a non-limiting example, the HCRTR1 protein of the present disclosure may comprise a sequence selected from:
i) SEQ ID NO:7;
ii) SEQ ID NO:8 and
iii) a sequence which is at least 85 % identical to SEQ ID NO:7 or 8.

In some embodiments, sequence ii) above is at least 90 % identical, at least 91 % identical, at least 92 % identical, at least 93 % identical, at least 94 % identical, at least 95 % identical, at least 96 % identical, at least 97 % identical, at least 98 % identical or at least 99 % identical to SEQ ID NO:7 or 8.

The above discussion about affinity ligands capable of selective interaction with the unglycosylated CNDP1 protein applies *mutatis mutandis* to affinity ligands capable of selective interaction with HCRTR1. That implies, for example, that the references to the CNDP1 fragments SEQ ID NO:1 and 2 are replaced by references to the HCRTR1 fragment SEQ ID NO:6.

### Examples

### 1. Generation of antigen

### a) Materials and methods

A suitable fragment of the target protein CNDP1 encoded by the EnsEMBL Gene ID ENSG00000150656 was selected using bioinformatic tools with the human genome sequence as template (Lindskog M et al (2005) Biotechniques 38:723-727, EnsEMBL, www.ensembl.org). The fragment was used as template for the production of a 102 amino acid long fragment corresponding to amino acids 32-133 (SEQ ID NO:1) of the CNDP1 protein (SEQ ID NO:3; EnsEMBL entry no. ENSP00000351682). Similarly, a second fragment was selected and used as template for the production of a 131 amino acid long fragment (SEQ ID NO:2) corresponding to amino acids 356-487 of the splice variant EnsEMBL entry no. ENSP00000351682 (SEQ ID NO:3), or amino acids 335-466 of the splice variant EnsEMBL entry no. ENSP00000416242 (SEQ ID NO:4).This second CNDP1 fragment covers one of the glycosylation sites.

A first fragment of the CNDP1 gene transcript containing nucleotides 321-627, of EnsEMBL entry number ENST00000358821 (SEQ ID NO:5), was isolated by a Superscript™ One-Step RT-PCR amplification kit with Platinum® Taq (Invitrogen) and a human total RNA pool panel as template (Human Total RNA, BD Biosciences Clontech). Flanking restriction sites Notl and Ascl were introduced into the fragment through the PCR amplification primers, to allow in-frame cloning into the expression vector (forward primer: CCGGCGCTGTTAGAGAAAG (SEQ ID NO:10), reverse primer: CAAGTGGCCGTAGAAGCAC (SEQ ID NO:11).

A second fragment of the CNDP1 gene transcript containing nucleotides 1296-1689, of EnsEMBL entry number ENST00000358821 (SEQ ID NO:5), or nucleotides 1237-1620 of EnsEMBL entry number ENST00000430634 (SEQ ID NO:16) was isolated by a Superscript™ One-Step RT-PCR amplification kit with Platinum® Taq (Invitrogen) and a human total RNA pool panel as template (Human Total RNA, BD Biosciences Clontech). Flanking restriction sites Notl and Ascl were introduced into the fragment through the PCR amplification primers, to allow in-frame cloning into the expression vector (forward primer: GATGAGCCTGGAACTAAAAC (SEQ ID NO:14), reverse primer: CCACCTGTTGATTTTCTCATTC (SEQ ID NO:15).

A suitable fragment of the target protein HCRTR1, encoded by the EnsEMBL Gene ID ENSG00000121764 was selected using bioinformatic tools with the human genome sequence as template (Lindskog M et al (2005) Biotechniques 38:723-727, EnsEMBL, www.ensembl.org). The fragment was used as templates for the production of a 48 amino acid long fragment corresponding to amino acids 1-48 (SEQ ID NO:6) of the HCRTR1 protein (SEQ ID NO:7; EnsEMBL entry no. ENSP00000362809, or SEQ ID NO:8; EnsEMBL entry no. ENSP00000362810).

For HCRTR1, a fragment of the HCRTR1 gene transcript containing nucleotides 1-144, of EnsEMBL entry number ENST00000373705 (SEQ ID NO:9), or nucleotides152-297 of EnsEMBL entry number ENST00000373706 (SEQ ID NO:17), was isolated by a Superscript™ One-Step RT-PCR amplification kit with Platinum® Taq (Invitrogen) and a human total RNA pool panel as template (Human Total RNA, BD Biosciences Clontech). Flanking restriction sites Notl and Ascl were introduced into the fragment through the PCR amplification primers, to allow in-frame cloning into the expression vector (forward primer: ATGGAGCCCTCAGCCACC (SEQ ID NO:12), reverse primer: GACCCACTCATACTGTTTTG (SEQ ID NO:13).

Then, the downstream primer was biotinylated to allow solid-phase cloning as previously described, and the resulting biotinylated PCR product was immobilized onto Dynabeads M280 Streptavidin (Dynal Biotech) (Larsson M et al (2000) J. Biotechnol. 80:143-157). The fragment was released from the solid support by Notl-Ascl digestion (New England Biolabs), ligated into the pAff8c vector (Larsson M *et al, supra)* in frame with a dual affinity tag consisting of a hexahistidyl tag for immobilized metal ion chromatography (IMAC) purification and an immunopotentiating albumin binding protein (ABP) from streptococcal protein G (Sjölander A et al (1997) J. Immunol. Methods 201:115-123; Ståhl S et al (1999) Encyclopedia of Bioprocess Technology: Fermentation, Biocatalysis and Bioseparation (Fleckinger MC and Drew SW, eds) John Wiley and Sons Inc., New York, pp 49-63), and transformed into *E*. *coli* BL21 (DE3) cells (Novagen). The sequences of the clones were verified by dye-terminator cycle sequencing of plasmid DNA amplified using TempliPhi DNA sequencing amplification kit (GE Healthcare, Uppsala, Sweden) according to the manufacturer's recommendations.

BL21 (DE3) cells harboring the expression vector were inoculated in 100 ml 30 g/l tryptic soy broth (Merck KGaA) supplemented with 5 g/l yeast extract (Merck KGaA) and 50 mg/l kanamycin (Sigma-Aldrich) by addition of 1 ml of an overnight culture in the same culture medium. The cell culture was incubated in a 1 liter shake flask at 37 °C and 150 rpm until the optical density at 600 nm reached 0.5-1.5. Protein expression was then induced by addition of isopropyl-β-D-thiogalactopyranoside (Apollo Scientific) to a final concentration of 1 mM, and the incubation was continued overnight at 25 °C and 150 rpm. The cells were harvested by centrifugation at 2400 g, and the pellet was re-suspended in 5 ml lysis buffer (7 M guanidine hydrochloride, 47 mM Na₂HPO₄, 2.65 mM NaH₂PO₄, 10 mM Tris-HCl, 100 mM NaCl, 20 mM β-mercaptoethanol; pH = 8.0) and incubated for 2 hours at 37 °C and 150 rpm. After centrifugation at 35300 g, the supernatant containing the denatured and solubilized protein was collected.

The His₆₋tagged fusion protein was purified by immobilized metal ion affinity chromatography (IMAC) on columns with 1 ml Talon® metal (Co²⁺) affinity resin (BD Biosciences Clontech) using an automated protein purification procedure (Steen J et al (2006) Protein Expr. Purif. 46:173-178) on an ASPEC XL4™ (Gilson). The resin was equilibrated with 20 ml denaturing washing buffer (6 M guanidine hydrochloride, 46.6 mM Na₂HPO₄, 3.4 mM NaH₂PO₄, 300 mM NaCl, pH 8.0-8.2). Clarified cell lysates were then added to the column. Thereafter, the resin was washed with a minimum of 31.5 ml washing buffer prior to elution in 2.5 ml elution buffer (6 M urea, 50 mM NaH₂PO₄, 100 mM NaCl, 30 mM acetic acid, 70 mM Na-acetate, pH 5.0). The eluted material was fractioned in three pools of 500, 700 and 1300 µl. The 700 µl fraction, containing the antigen, and the pooled 500 and 1300 µl fractions were stored for further use.

The antigen fraction was diluted to a final concentration of 1 M urea with phosphate buffered saline (PBS; 1.9 mM NaH₂PO₄, 8.1 mM Na₂HPO₄, 154 mM NaCl) followed by a concentration step to increase the protein concentration using Vivapore 10/20 ml concentrator with molecular weight cut off at 7500 Da (Vivascience AG). The protein concentration was determined using a bicinchoninic acid (BCA) micro assay protocol (Pierce) with a bovine serum albumin standard according to the manufacturer's recommendations. The protein quality was analyzed on a Bioanalyzer instrument using the Protein 50 or 200 assay (Agilent Technologies).

### b) Results

Gene fragments corresponding to amino acid SEQ ID NO:1, SEQ ID NO:2 and SEQ ID NO:6 was successfully isolated by RT-PCR from a human RNA pool using specific primers. The fragments code for amino acids 32 to 133 and amino acids 356-487 (335-466) of the target protein CNDP1 (SEQ ID NO:3 (SEQ ID NO:4)), and amino acids 1 to 48 of the target protein HCRTR1 (SEQ ID NO:7 or SEQ ID NO:8), respectively. The 102, 131 and 48 amino acid long fragments (SEQ ID NO:1, SEQ ID NO:2 and SEQ ID NO:6) of the target proteins were designed to lack transmembrane regions to ensure efficient expression in *E. coli,* and to lack any signal peptide, since those are cleaved off in the mature protein. In addition, the protein fragments were designed to consist of a unique sequence with low homology with other human proteins, to minimize cross reactivity of generated affinity reagents, and to be of a suitable size to allow the formation of conformational epitopes and still allow efficient cloning and expression in bacterial systems.

Clones encoding the correct amino acid sequences were identified, and, upon expression in *E. coli,* protein fragments of the correct size were produced and subsequently purified using immobilized metal ion chromatography. Samples were diluted to a final concentration of 1 M urea and concentration of the samples to 1 ml. The concentration of the protein fragments was determined: CNDP1, first fragment (102 amino acids, SEQ ID NO:1): 11.2 mg/ml and 100.0 % pure. CNDP1, second fragment (131 amino acids, SEQ ID NO:2): 25.4 mg/ml and 100.0 % pure. HCRTR1 (48 amino acids, SEQ ID NO:6): 34.9 mg/ml and 96.0 % pure.

### 2. Generation of antibodies

### a) Materials and methods

The purified CNDP1 and HCRTR1 fragments (SEQ ID NO:1, SEQ ID NO:2 and SEQ ID NO:6) as obtained above were used as antigens to immunize rabbits in accordance with the national guidelines (Swedish permit no. A 84-02). Rabbits were immunized intramuscularly with 200 µg of antigen in Freund's complete adjuvant as the primary immunization, and boosted three times in four week intervals with 100 µg antigen in Freund's incomplete adjuvant.

Antisera from the immunized animals were purified by a three-step immunoaffinity based protocol (Agaton C et al (2004) J. Chromatogr. A 1043:33-40; Nilsson P et al (2005) Proteomics 5:4327-4337). In the first step, 7 ml of total antiserum was buffered with 10x PBS to a final concentration of 1x PBS (1.9 mM NaH₂PO₄, 8.1 mM Na₂HPO₄, 154 mM NaCl), filtered using a 0.45 µm pore-size filter (Acrodisc®, Life Science) and applied to an affinity column containing 5 ml N-hydroxysuccinimide-activated Sepharose™ 4 Fast Flow (GE Healthcare) coupled to the dual affinity tag protein His₆-ABP (a hexahistidyl tag and an albumin binding protein tag) expressed from the pAff8c vector and purified in the same way as described above for the antigen protein fragment. In the second step, the flow-through, depleted of antibodies against the dual affinity tag His₆-ABP, was loaded at a flow rate of 0.5 ml/min on a 1 ml Hi-Trap NHS-activated HP column (GE Healthcare) coupled with the CNDP1 or HCRTR1 protein fragments used as antigen for immunization (SEQ ID NO:1, SEQ ID NO:2 and SEQ ID NO:6). The His₆-ABP protein and the protein fragment antigens were coupled to the NHS activated matrix as recommended by the manufacturer. Unbound material was washed away with 1x PBST (1x PBS, 0.1 % Tween20, pH 7.25), and captured antibodies were eluted using a low pH glycine buffer (0.2 M glycine, 1 mM EGTA, pH 2.5). The eluted antibody fraction was collected automatically, and loaded onto two 5 ml HiTrap™ desalting columns (GE Healthcare) connected in series for efficient buffer exchange in the third step. The second and third purification steps were run on the ÄKTAxpress™ platform (GE Healthcare). The antigen selective (mono-specific) antibodies (msAbs) were eluted with PBS buffer, supplemented with glycerol and NaN₃ to final concentrations of 40 % and 0.02 %, respectively, for long term storage at -20 °C (Nilsson P et al (2005) Proteomics 5:4327-4337).

The specificity and selectivity of the affinity purified antibody fraction were analyzed by binding analysis against the antigen itself and against 94 other human protein fragments in a protein array set-up (Nilsson P et al (2005) Proteomics 5:4327-4337). The protein fragments were diluted to 40 µg/ml in 0.1 M urea and 1x PBS (pH 7.4) and 50 µl of each were transferred to the wells of a 96-well spotting plate. The protein fragments were spotted in duplicate and immobilized onto epoxy slides (SuperEpoxy, TeleChem) using a pin-and-ring arrayer (Affymetrix 427). The slide was washed in 1x PBS (5 min) and the surface was then blocked (SuperBlock®, Pierce) for 30 minutes. An adhesive 16-well silicone mask (Schleicher & Schuell) was applied to the glass before the mono-specific antibodies were added (diluted 1:2000 in 1x PBST to appr. 50 ng/ml) and incubated on a shaker for 60 min. Affinity tag-specific IgY antibodies were co-incubated with the mono-specific antibodies in order to quantify the amount of protein in each spot. The slide was washed with 1x PBST and 1x PBS twice for 10 min each. Secondary antibodies (goat anti-rabbit antibody conjugated with Alexa 647 and goat anti-chicken antibody conjugated with Alexa 555, Molecular Probes) were diluted 1:60000 to 30 ng/ml in 1x PBST and incubated for 60 min. After the same washing procedure, as for the first incubation, the slide was spun dry and scanned (G2565BA array scanner, Agilent), thereafter images were quantified using image analysis software (GenePix 5.1, Axon Instruments).

In addition, the specificity and selectivity of the affinity-purified antibody were analyzed by Western blot. Western blot was performed by separation of total protein extracts from selected human cell lines on pre-cast 10-20 % SDS-PAGE gradient gels (Bio-Rad Laboratories) under reducing conditions, followed by electro-transfer to PVDF membranes (Bio-Rad Laboratories) according to the manufacturer's recommendations. The membranes were blocked (5 % dry milk, 1x TBST; 0.1 M Tris-HCl, 0.5 M NaCl, 0.1 % Tween20) for 1 h at room temperature, incubated with the primary affinity purified antibody (diluted 1:500 in blocking buffer) and washed in TBST. The secondary HRP-conjugated antibody (swine anti-rabbit immunoglobulin/HRP, DakoCytomation) was diluted 1:3000 in blocking buffer and chemiluminescence detection was carried out using a Chemidoc™ CCD camera (Bio-Rad Laboratories) and SuperSignal® West Dura Extended Duration substrate (Pierce), according to the manufacturer's protocol.

### b) Results

The quality of polyclonal antibody preparations has proven to be dependent on the degree of stringency in the antibody purifications, and it has previously been shown that depletion of antibodies directed against epitopes not originated from the target protein is necessary to avoid cross-reactivity to other proteins and background binding (Agaton C et al (2004) J. Chromatogr. A 1043:33-40). Thus, a protein microarray analysis was performed to ensure that mono-specific polyclonal antibodies of high specificity had been generated by depletion of antibodies directed against the His₆-tag as well as of antibodies against the ABP-tag.

To quantify the amount of protein in each spot of the protein array, a two-color dye labeling system was used, with a combination of primary and secondary antibodies. Tag-specific IgY antibodies generated in hen were detected with a secondary goat anti-hen antibody labeled with Alexa 555 fluorescent dye. The specific binding of the rabbit msAb to its antigen on the array was detected with a fluorescently Alexa 647 labeled goat anti-rabbit antibody. Each protein fragment was spotted in duplicates. The protein array analysis shows that the affinity purified mono-specific antibodies against CNDP1 and HCRTR1 are highly selective to the correct protein fragment and has a very low background to all other protein fragments analyzed on the array.

The result of the Western blot analysis for first CNDP1 mono-specific antibody shows that the antibody distinctly detects two bands of approximately 190 kDa and approximately 60 kDa in plasma. The predicted molecular weight of CNDP1 is 57 kDa (as calculated from the CNDP1 amino acid sequence SEQ ID NO:3), corresponding well to one of the detected bands. The other band at 190 kDa can refer to a predicted glycosylated isoform of the CNDP1 protein.

The result of the Western blot analysis for the second CNDP1 mono-specific antibody shows that the antibody distinctly detects one band of approximately 60 kDa in plasma. The predicted molecular weight of CNDP1 should be somewhere between 54-57 kDa (as calculated from the CNDP1 amino acid sequence SEQ ID NO:3 and SEQ ID NO:4), corresponding well to the detected band.

The result of the Western blot analysis for HCRTR1 shows that the HCRTR1 antibody reveals a single band of approximately 50 kDa in plasma, with no other bands detected. The predicted molecular weight of HCRTR1 should be some where between 44-48 kDa (as calculated from the HCRTR1 amino acid sequence SEQ ID NO:7 and SEQ ID NO:8), corresponding well to the results obtained.

### 3. Heat sensitive antigen retrieval and protein detection

Antibodies, obtained as described in Example 2, were coupled to carboxylated beads (COOH Microspheres, Luminex-Corp.) in accordance to the manufacturer's protocol and all antibody-coupled beads were counted using the LX200 instrumentation (Luminex-Corp.). The coupling efficiency for each antibody was determined via R-Phycoerythrin labelled anti-rabbit IgG antibody (Jackson lmmunoResearch) and a 100-plex bead mixture was created as previously described (Schwenk J et al (2007) Mol Cell Proteomics 6(1):125-32).

Serum and plasma samples were thawed at RT and centrifuged for 10 min at 10,000 rpm and transferred into a microtiter plate (Abgene). The plates were centrifuged (1 min at 3,000 rpm) and 3 pl of each sample was added to 24.5 µl 1 × PBS with a liquid handler (Plate mate 2x2, Matrix). N-hydroxysuccinimidyl ester of biotinoyl-tetraoxapentadecanoic acid (NHS-PE04-Biotin, Pierce) was then added at 10-fold molar excess to yield an overall 1/10 sample dilution followed by an incubation over 2 h at 4°C in a microtiter plate shaker (Thermomixer, Eppendorf). The reaction was stopped by the addition of a 250-fold molar excess of Tris-HCl, pH 8.0 over biotin and incubated for another 20 min at 4°C prior to a final storage at -80°C.

All samples were subsequently utilized without removing unincorporated biotin and diluted 1/50 (Plate Mate, Matrix Corp.) in an assay buffer composed of 0.5% (w/v) polyvinylalcohol and 0.8% (w/v) polyvinylpyrrolidone (Sigma) in 0.1% casein in PBS (PVXC) supplemented with 0.5 mg/ml non-specific rabbit IgG (Bethyl). All antibodies were tested on samples heat treated in a thermocycler for 30 min at 23°C, 56°C, or for 15 min at 72°C. Then 45 µl were added to 5 µl of bead mixtures in a filter-bottomed microtiter plate (Millipore) and incubation took place over night on a shaker at an ambient temperature. Beads were washed in wells with 3x 50 µl PBST (1 × PBS pH 7.4, 0.1 % Tween20) on a vacuum device (Millipore) followed by 10 min with 50 µl of a stop solution containing 0.1% paraformaldehyde in PBS. Beads were washed 1 × 50 µl PBST and 30 µl of 0.5 µg/ml R-Phycoerythrin labelled streptavidin (Invitrogen) in PBST were added and incubated for 20 min. Finally, wells were washed 3 × 50 µl and measured in 150 µl PBST.

### 4. Selection of antibodies to assay prostate cancer patients

Antibodies validated within the frame-work of the Human Protein Atlas (HPA) program were used in the experiment. The criteria for the selection of antibodies were a successful specificity and cross-reactivity analysis of using an antigen microarray composed of 384 unique PrEST proteins and an antibody concentration of more than 50 µg/ml after affinity capture from the polyclonal sera. Antibody solutions with a concentration less than 50 µg/ml were shown to yield significantly lower fractions of proteins to be detected above the noise of the assay. A further selection was based on Western blot analysis, harvesting only antibodies that showed a single band at the expected protein molecular weight (+ 20%) tested with a single plasma sample, and 441 antibodies were found. In this list, antibodies were additionally sorted according to their Western blot results for cell lines and tissue lysates, as well as intensity levels observed during the specificity analysis on protein arrays. From these, 96 antibodies were chosen for detection of interesting prostate cancer biomarkers.

### 5. Discovery cohort

### a) Materials and methods

From Malmö, University Hospital Sweden, 84 plasma samples donated from anonymized individuals were obtained. From these, total PSA values (tPSA) and free-to-total PSA ratios (f/tPSA) had been determined during clinical routine analysis. The patients were then grouped into four groups (A-D) based on these clinical parameters (Table 1). High PSA-values (tPSA) correlates with an increased risk of prostate cancer, and also with tumor aggressiveness. In cases with a minor elevation of tPSA, the free-to-total PSA (f/tPSA) might be indicative, with a lower ratio correlating with an increased risk of prostate cancer.

**Table 1**

| Group | n | tPSA [ng/ml] | f/tPSA |
|---|---|---|---|
| A | 20 | 565 (61 - 3030) | - |
| B | 23 | 0.9 (0.5 - 1.5) | - |
| C | 21 | 5.5 (2.1 - 9.4) | ≤ 0.15 |
| D | 20 | 4.1 (2.0 - 9.0) | ≥ 0.30 |

The 96 antibodies, obtained and chosen as described in Examples 2 and 4 were applied to the plasma samples according to methods described in Example 3. For statistical analyses, a linear model (ImFit) of the limma package (Smyth, G.K. (2004) Stat Appl Genet Mol Biol 3:Article 3) was used to calculate P-values for a comparative analysis (P ≤ 0.01 was considered significant), and standard errors were moderated using a simple empirical Bayes model to compute moderated t-statistics for each comparison and for each antibody.

### b) Results

The protein profiles of the patient groups A-D were compared, and a number of potential biomarker candidates were identified (Table 2). Almost all were found to differ between groups at one of three retrieval temperatures, and one of the proteins (CNDP1, detected using the antibody directed against SEQ ID NO:1) differed between groups at two temperatures, both 23°C and 56°C. For both CNDP1 (Figure 2A) and HCRTR1 (Figure 2B) there was a significant difference in protein expression between patient group A (high risk of prostate cancer) and patient group B (low risk of prostate cancer) at 56°C.

**Table 2**

| **Temperature** | **Gene Name** | ***P*-Value** | **Comparison** |
|---|---|---|---|
| 23°C | FLNB | 1.5 × 10⁻⁴ | A - B |
| | CNDP1 | 1.8 × 10⁻⁴ | A - B |
| | UBQLN2 | 2.2 × 10⁻³ | A - B |
| | MID1 | 7.0 × 10⁻³ | A - B |
| | NUCB2 | 8.6 × 10⁻³ | A - B |
| | CNDP1 | 4.3 × 10⁻⁵ | A - C |
| | EPHX3 | 2.4 × 10⁻³ | A - C |
| | CNDP1 | 9.9 × 10⁻⁴ | A - D |
| | UBQLN2 | 5.4 × 10⁻³ | A - D |
| | FOXD4L4 | 9.8 × 10⁻³ | A - D |
| 56°C | ALPL | 8.2 × 10⁻³ | A - B |
| | CNDP1 | 2.2 × 10⁻⁴ | A - B |
| | HCRTR1 | 9.5 × 10⁻⁴ | A - B |
| | CNDP1 | 4.9 × 10⁻³ | A - C |
| | FAM82A2 | 6.6 × 10⁻³ | A - C |
| | FMO5 | 9.7 × 10⁻³ | A - C |
| | GHRL | 9.3 × 10⁻³ | A - C |
| | HCRTR1 | 5.5 × 10⁻⁴ | A - C |
| | CALHM2 | 9.7 × 10⁻⁴ | A - C |
| | CNDP1 | 1.4 × 10⁻³ | A - D |
| | CALHM2 | 1.4 × 10⁻³ | A - D |
| | SLC29A1 | 7.4 × 10⁻³ | B - C |
| | FBXL6 | 3.1 × 10⁻³ | B - D |
| 72°C | ATRN | 5.0 × 10⁻³ | A-B |
| | TMEM59L | 1.9 × 10⁻³ | A - B |
| | TMEM59L | 4.1 × 10⁻³ | A - C |
| | FBXL6 | 8.3 × 10⁻³ | B - C |
| | NPM1 | 1.7 × 10⁻³ | B - C |
| | GRAMD1A | 2.6 × 10⁻³ | B - C |
| | DNAJB11 | 8.7 × 10⁻³ | B - D |

### 6. Technical validation of the potential biomarkers by Western blot

### a) Materials and methods

A technical validation of the CNDP1 and HCRTR1 biomarkers was performed by Western blot analysis. Three individual patient plasma samples from each group were depleted from IgG and serum albumin by the use of Affibody molecules (Affibody) coupled to SulfoLink matrix (Pierce). Samples were diluted 1/10 in PBS and the flow through was applied per lane to SDS-gel separation (4-12% BisTris, Invitrogen) followed by transfer onto PVDF membranes (Invitrogen). The transfer was confirmed by staining with Ponceau (Pierce), and blots were blocked in 5% milk powder (Semper) in TBST for 60 min. Polyclonal antibodies (obtained as described in Example 2) directed against the CNDP1 protein fragment SEQ ID NO:1 and the HCRTR1 protein protein fragment SEQ ID NO:6 were incubated at optimized concentrations over night at 4°C in the milk powder buffer. All membranes were washed in TBST 3x 5 min and detection was performed using HRP labelled anti-rabbit antibody (Dako) and a chemiluminescent substrate (Immobilon, Millipore).

### b) Results

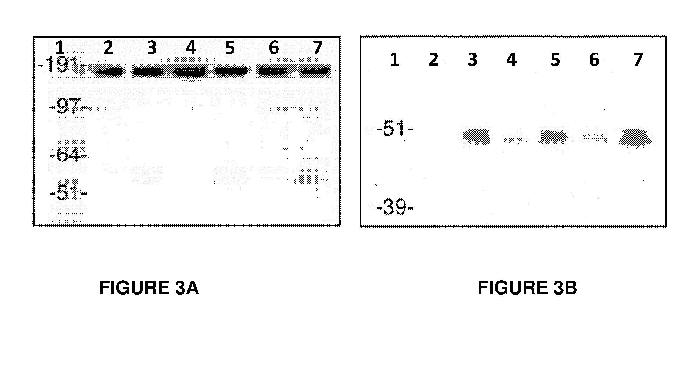

The analysis of HCRTR1 showed a very clear difference between the two groups with a strong band in the right size (48 kDa) in all three plasma samples from patients in group B, while much less protein is observed for plasma from group A (Figure 3B). For CNDP1, discovered both at the 23°C and 56°C retrieval temperatures, an interesting pattern was observed. The presumed glycosylated form of this protein with a size of 190 kDa is present in similar amounts for all six samples analyzed. In contrast, a weaker band from the possibly unglycosylated isofrom of the protein can be observed (57 kDa) and this band is differentially present between the two sample groups (Figure 3A). The Western blot analysis thus suggests that the antibody binds to both the glycosylated and the unglycosylated form of the CNDP1 protein in the Western blot setting, and that more of the glycosylated form than the unglycosylated form was present in the sample. There was a clear difference in protein expression between the two patient groups, A and B, representing less and more aggressive prostate cancer, respectively. It was shown that the amount of unglycosylated CNDP1 protein differed between the two patient groups, with a lower amount of unglycosylated CNDP1 protein in samples from patients with a more aggressive form of prostate cancer. This indicates that only the unglycosylated form of the CNDP1 protein was detected in the high-throughput bead array assay, described in Example 3, used in the discovery cohort (Example 5).

### 7. Western blot analyses of captured protein

### a) Materials and methods

Immuno-capture analysis was performed using the protocol and beads employed in the bead array assays on samples from the two patient groups, A and B, representing less and more aggressive prostate cancer characteristics, respectively. The proteins were eluted and detected using Western blot, performed as described in Example 6 above, with the following exception: The proteins were either detected using (i) a streptavidin-HRP conjugate, or (ii) an anti-CNDP1 antibody, obtained using the CNDP1 fragment SEQ ID NO:1 as described in Example 2.

### b) Results

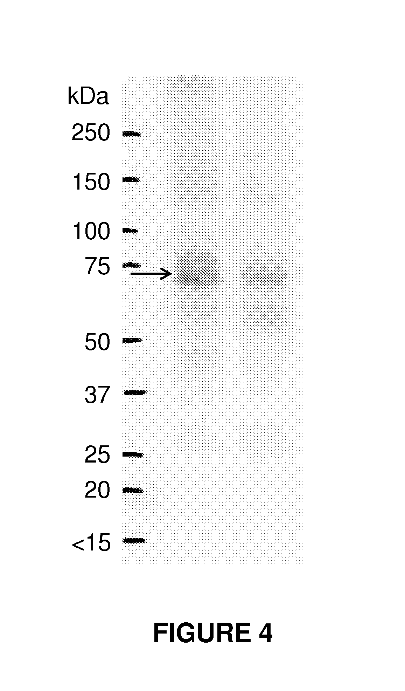

Using a streptavidin-HRP conjugate for detection, a single band migrating around 60kD was detected (Figure 4). This band most likely corresponds to CNDP1, and was present at a higher level in the sample representing the less aggressive prostate cancer group. The result was confirmed in a second western blot experiment using an anti-CNDP1 antibody, obtained using the CNDP1 fragment SEQ ID NO:1 as described in Example 2, which detects a single protein migrating around 60kD. No other bands could be detected, indicating that CNDP1 is not part of a multi-protein complex. Furthermore, the lack of the 190 kDa form of CNDP1 demonstrates that the unglycosylated form of CNDP1 is exclusively detected in the bead array assay.

### 8. Validation cohort

### a) Materials and methods

Biobank plasma samples were obtained, either from patients diagnosed with aggressive prostate cancer, patients diagnosed with less aggressive prostate cancer, or disease-free control subjects. All included in the study gave written informed consent, and were part of the Cancer Prostate in Sweden-study performed at Umeå University. In total, 90 plasma samples were used, 30 in each group. Aggressive prostate cancer was defined as either tumor stage T3/T4, N1, Gleason sum ≥ 8, or PSA > 50 ng/ml. Less aggressive prostate cancer was defined as tumor stage T1/T2 and NO/NX or MO/MX, PSA < 50 ng/ml, and Gleason sum 4-7. Polyclonal antibodies obtained as described in Example 2 were applied to the plasma samples according to methods described in Example 3 (the anti-CNDP1 antibody was obtained using the CNDP1 fragment SEQ ID NO:1). For statistical analyses, profiles were age-normalized with a linear regression model. The calculated residual values were compared in Mann-Whitney tests to reveal P-values (P ≤ 0.05 was considered significant).

### b) Results

The unglycosylated CNDP1 protein was confirmed as a biomarker in the validation cohort. The level of unglycosylated CNDP1 protein was significantly lower in the more aggressive cases of prostate cancer than in the less aggressive or control cases (Figure 5). For the HCRTR1 protein, there was no significant difference in protein level between the groups studied. However, this could be due to factors such as the plasma sample preparation (EDTA vs Heparin), time of sample storage (Biobank vs clinical material obtained purely for this study), the cohort selection and the connection to tPSA (greater tPSA differences in the discovery cohort).

### 9. Protein detection using the sandwich immunoassay

### a) Materials and methods

Plasma samples from prostate cancer patients were treated similarly to the direct labeling procedure as described in Example 3, but utilized without biotinylation. Samples were thawed at room temperature, diluted 1/10 in PBS followed by 1/50 in PVXCas buffer and heat treated for 30 min at 56°C. Beads coupled with anti-CNDP1 antibody, selective for the unglycosylated form of the protein as shown in Example 7 above, and obtained as described in Example 2, were incubated overnight together with the samples under permanent shaking. The beads were then washed 3x 100 µl PBST. Another anti-CNDP1 antibody (AF2489, goat IgG, R&D Systems) was biotinylated using a 50x molar excess of PE04-biotin (Pierce) over antibody, and was diluted as detection reagent to 0.4 µg/ml. After 60 min incubation, the beads were washed as described above, and Phycoerythrin-modified streptavidin 0.6 µg/ml in PBST was added and incubated for 20 min with the beads.

Following a final washing step the beads were measured in the LX200 instrumentation in 100 µl PBST.

### b) Results

Samples from patients classified with less aggressive and aggressive prostate cancer were analyzed using the sandwich immunoassay. The anti-CNDP1 polyclonal antibody, obtained using the CNDP1 fragment SEQ ID NO:1 as described in Example 2, was used as capture antibody and a goat anti-CNDP1 antibody, AF2489, was used as detection reagent. The resulting CNDP1 profiles show a low variability (less than 7%), and the same trend as the direct labeling approach is observed. Patients with aggressive prostate cancer have lower levels of unglycosylated CNDP1 than patients with less aggressive prostate cancer (Figure 6). Due to the added specificity from the detection antibody, it was confirmed that the unglycosylated CNDP1 protein allows the two patient groups to be separated.

In conclusion, the results from this sandwich assay, which is an assay frequently applied in a clinical setting, shows that the unglycosylated CNDP1 protein is a biomarker for prostate cancer that can be detected using established routine assays.

## Claims

1. Method of determining whether a mammalian subject belongs to a first or a second group of subjects, wherein the risk of having a prostate cancer, such as an aggressive prostate cancer, is higher in the first group than in the second group, comprising the steps of:
a) evaluating an amount of unglycosylated CNDP1 protein in a sample derived from blood, semen or urine from the subject and determining a sample value corresponding to the evaluated amount;
b) comparing the sample value with a predetermined reference value; and if the sample value is lower than or equal to the reference value,
c1) concluding that the subject belongs to the first group; and if the sample value is higher than the reference value,
c2) concluding that the subject belongs to the second group.

2. Method of determining whether a mammalian subject belongs to a first or a second group of subjects, wherein the risk of having a prostate cancer, such as an aggressive prostate cancer, is higher in the first group than in the second group, comprising the steps of:
c) quantifying the degree of glycosylation of CNDP1 protein in a sample derived from blood, semen or urine from the subject;
a) comparing the degree of glycosylation of step a) with a predetermined reference degree of glycosylation; and if the degree of glycosylation of step a) is higher than or equal to the reference,
c1) concluding that the subject belongs to the first group; and if the degree of glycosylation of step a) is lower than the reference,
c2) concluding that the subject belongs to the second group.

3. Method according to claim 1 or 2, wherein an aggressive prostate cancer is defined as a metastasizing prostate cancer, a prostate cancer showing a Gleason sum of ≥ 8 and/or a prostate cancer from a subject having a PSA level of above 50 ng/ml.

4. Method of detecting a prostate cancer in a mammalian subject, comprising the step of:
a) evaluating an amount of unglycosylated CNDP1 protein in a sample derived from blood, semen or urine from the subject and determining a sample value corresponding to the evaluated amount;
b) comparing the sample value with a predetermined reference value; and if the sample value is lower than or equal to the reference value,
c) examining at least one prostate tissue biopsy from the subject for the presence of tumor cells.

5. Method of selecting a mammalian subject for a prostate tissue examination, comprising the steps of:
a) evaluating an amount of unglycosylated CNDP1 protein in a sample derived from blood, semen or urine from the subject and determining a sample value corresponding to the evaluated amount;
b) comparing the sample value with a predetermined reference value; and if the sample value is lower than or equal to the reference value,
c) selecting the subject for the prostate tissue examination.

6. Method for determining whether a mammalian subject, such as a mammalian subject having prostate cancer, belongs to a first or a second group, wherein the prognosis of subjects of the first group is worse than the prognosis of subjects of the second group, comprising the steps of:
a) evaluating an amount of unglycosylated CNDP1 protein in a sample derived from blood, semen or urine from the subject and determining a sample value corresponding to the evaluated amount;
b) comparing the sample value with a predetermined reference value; and if the sample value is lower than or equal to the reference value,
c1) concluding that the subject belongs to the first group; and if the sample value is higher than the reference value,
c2) concluding that the subject belongs to the second group.

7. Method for determining whether a mammalian subject is not in need of a prostate cancer treatment regimen, comprising the steps of:
a) evaluating an amount of unglycosylated CNDP1 protein in a sample derived from blood, semen or urine from the subject and determining a sample value corresponding to the evaluated amount;
b) comparing the sample value with a predetermined reference value; and if the sample value is higher than the reference value,
c) concluding that the subject is not in need of the prostate cancer treatment regimen.

8. Non-treatment strategy method for a mammalian subject, comprising the steps of:
a) evaluating an amount of unglycosylated CNDP1 protein in a sample derived from blood, semen or urine from the subject and determining a sample value corresponding to the evaluated amount;
b) comparing the sample value with a predetermined reference value; and if the sample value is higher than the reference value,
c) refraining from treating the subject with a prostate cancer treatment regimen.

9. Method according to any one of the preceding claims, wherein the sample is an optionally diluted serum or plasma sample.

10. CNDP1 protein fragment which consists of 150 amino acids or less, such as 135 amino acids or less, and comprises or consists of amino acid sequence SEQ ID NO:1 or 2.

11. Use *ex vivo* of an unglycosylated CNDP1 protein as a diagnostic marker for prostate cancer.

12. Use of an unglycosylated CNDP1 protein, or an antigenically active fragment thereof,
*ex vivo* for the selection or purification or
*in vivo* for the production
of a diagnostic agent for prostate cancer, such as aggressive prostate cancer.

13. Affinity ligand capable of selective interaction with an unglycosylated CNDP1 protein fragment consisting of amino acid sequence SEQ ID NO:1 or 2.

14. Affinity ligand capable of selective interaction with an unglycosylated CNDP1 protein, which is immobilized onto a solid support suitable for a sandwich assay or bound to a bead.

15. Use *ex vivo* of an affinity ligand capable of selective interaction with an unglycosylated CNDP1 protein as a diagnostic agent for prostate cancer, such as aggressive prostate cancer.

16. Kit comprising:
a) a first affinity ligand capable of selective interaction with an unglycosylated CNDP1 protein;
b) a quantifiable second affinity ligand capable of interaction with the unglycosylated CNDP1 protein when bound to the first affinity ligand; and optionally
c) a solid support on which the first affinity ligand is immobilized or which is prepared for immobilization of the first affinity ligand.
